# EUROPEAN PATENT SPECIFICATION

(11) **EP 4 403 108 B1**
(45) Date of publication and mention of the grant of the patent: **09.12.2026**
(21) Application number: 22885173.9
(22) Date of filing: 30.06.2022
(51) Int. Cl.: A61B 5/366, A61B 5/318, A61B 5/316, A61B 5/349, A61B 5/352

(54) **APPARATUS, COMPUTER DEVICE AND COMPUTER-READABLE STORAGE MEDIUM FOR ANALYZING HIGH-FREQUENCY QRS-COMPLEX DATA**
VORRICHTUNG, COMPUTERVORRICHTUNG UND COMPUTERLESBARES SPEICHERMEDIUM ZUR ANALYSE VON HOCHFREQUENZ-QRS-KOMPLEXDATEN
APPAREIL, DISPOSITIF INFORMATIQUE ET SUPPORT DE STOCKAGE LISIBLE PAR ORDINATEUR POUR L'ANALYSE DE DONNÉES DE COMPLEXES QRS À HAUTE FRÉQUENCE

(30) Priority: 01.11.2021 CN 202111281766
(43) Date of publication of application: 24.07.2024
(73) Proprietor: Hyperbio Biological Technology Co., Ltd, Changsha, Hunan 410000 (CN)
(72) Inventor: LI, Xiaoqin, Changsha, Hunan 410000 (CN); HUANG, Qingxi, Changsha, Hunan 410000 (CN); HUANG, Qinghong, Changsha, Hunan 410000 (CN)
(74) Representative: Ström & Gulliksson AB
(86) International application number: PCT/CN2022/102597
(87) International publication number: WO 2023/071268

(56) References cited:
- CN-A- 101 014 283
- CN-A- 101 522 099
- CN-A- 104 586 383
- CN-A- 110 477 906
- CN-A- 111 419 212
- CN-A- 113 712 569
- US-A1- 2004 039 292
- US-A1- 2004 039 292
- US-A1- 2009 318 820
- US-B1- 7 539 535
- BALFOUR PELBRETON C ET AL: "High-frequency QRS analysis to supplement ST evaluation in exercise stress electrocardiography: Incremental diagnostic accuracy and net reclassification", JOURNAL OF NUCLEAR CARDIOLOGY, vol. 27, no. 6, 30 November 2018 (2018-11-30), pages 2063 - 2075, XP037318066, ISSN: 1071-3581, DOI: 10.1007/S12350-018-01530-W
- THOMAS GREGORY S ET AL: "An opportunity to improve exercise test accuracy by incorporating high frequency QRS (HFQRS) waveform assessment", JOURNAL OF NUCLEAR CARDIOLOGY, vol. 27, no. 6, 28 January 2019 (2019-01-28), pages 2076 - 2079, XP037318087, ISSN: 1071-3581, DOI: 10.1007/S12350-019-01600-7
- TAFRESHI REZA ET AL: "Automated analysis of ECG waveforms with atypical QRS complex morphologies", BIOMEDICAL SIGNAL PROCESSING AND CONTROL, vol. 10, 1 March 2014 (2014-03-01), NL, pages 41 - 49, XP093211360, ISSN: 1746-8094, DOI: 10.1016/j.bspc.2013.12.007

## Description

### Field of the Invention

The present application relates to the technical field of medical instruments, and in particular to a method and apparatus for analyzing high-frequency QRS-complex data.

### Background of the Invention

Electrocardiogram (ECG) describes the electrical activity of the muscle groups that make up the different ventricles of the heart, electrocardiogram (ECG) signals are recordings of body surface electrode or implantable electrode signal data, the recording of the electrode signal data indicates body potential changes due to the extended electrical activity within the heart, the electrocardiogram (ECG) is a vector, different positions of the heart are distributed differently, and a rate at which the current signal passes through different parts of the body is different, whereby the electrocardiogram (ECG) signals distinguishing different parts of the heart can be acquired, and the electrocardiogram (ECG) signals of different parts of the heart are analyzed and processed to obtain cardiac health information of the patient.

In the prior art, information about cardiac activity is detected and analyzed by electrocardiogram (ECG), and the information is focused on the P-QRS-T segment, and in particular, according to the data of the QRS segment, the pattern is determined to become longer or abnormal so as to determine the presence of pathology in cardiac activity. Generally, the main frequency range of electrocardiogram (ECG) signals is 0.05Hz-100Hz, and relevant information of many common cardioactive pathologies can be obtained in this frequency range, but ECG signals at a high frequency range greater than 100Hz can find more valuable information about cardioactive pathologies.

US 2004/039292 discloses a system for the diagnosis and monitoring of coronary artery disease, acute coronary syndromes, cardiomyopathy and other cardiac conditions using cardiac electrical data from a patient where it employs real-time calculations to identify reduced amplitude zones (RAZ) and kurtosis. XP037318066 discloses analysing HF-QRS data, a lead positive index computation and identifies the amplitude reduction in the dynamic HF-QRS as a biomarker in exercise versus recovery.

However, existing analytical processing of high-frequency QRS segment data has only been studied in some fields (as in the paper High-Frequency Electrocardiogram Analysis of the Entire QRS in the Diagnosis and Assessment of Coronary Artery Disease. Abbound et al., Progress in Cardiovascular Diseases journal, Vol. XXXV, No. 5, March/April 1993) and cannot be generalized for wider application thereof.

Accordingly, it is necessary to provide a method and apparatus for analyzing high-frequency QRS-complex data that is capable of improving doctor's electrocardiogram reading efficiency in view of the above-mentioned technical problems.

The invention is defined by the independent claims, preferred embodiments are defined in the dependent claims. The present application discloses a method for analyzing high-frequency QRS-complex data, the method comprises:
acquiring high-frequency QRS waveform data, which is output by means of at least one electrocardiogram lead;
calculating an amplitude decrease relative value and the absolute value of an amplitude according to the high-frequency QRS waveform data and by means of a first function, so as to form a lead positive index;
calculating a waveform change shape index according to the high-frequency QRS waveform data and by means of a second function, wherein the waveform change shape index is used for indicating a shape category to which a waveform change belongs; and
performing analysis according to the lead positive index and the waveform change shape index and by means of a second preset rule, so as to label an attention level corresponding to the high-frequency QRS waveform data.

In one embodiment, after calculating the amplitude decrease relative value and the absolute value of the amplitude according to the high-frequency QRS waveform data and by means of the first function, so as to form the lead positive index, the method further includes: dividing a positive position into a first category or a second category according to a first preset rule based on the kind and quantity of the high-frequency QRS waveform data indicated as positive by the lead positive index; performing analysis according to the lead positive index and the waveform change shape index and by means of the second preset rule, so as to label the attention level corresponding to the high-frequency QRS waveform data, includes: performing analysis according to the lead positive index, the waveform change shape index, and the divided category of the positive position and by means of the second preset rule, so as to label the attention level corresponding to the high-frequency QRS waveform data.

In one embodiment, acquiring the high-frequency QRS waveform data, which is output by means of at least one electrocardiogram lead, includes: acquiring high-frequency QRS waveform data for at least one sampling period, which is outputted by means of at least one electrocardiogram chest lead and/or limb lead, the high-frequency QRS waveform data being obtained by extracting data in an interval of 150HZ-250HZ from a current signal acquired by an electrode pad applied to a human body.

In one embodiment, calculating the amplitude decrease relative value and the absolute value of the amplitude according to the high-frequency QRS waveform data and by means of the first function, so as to form the lead positive index, includes: intercepting exercise-time-period waveform data from the high-frequency QRS waveform data, choosing data at which the RMS voltage value is the largest in the exercise-time-period waveform data as a first reference point, and choosing a point at which the RMS voltage value is the smallest after the time of the first reference point as a second reference point; and calculating a difference between the RMS voltage value of the first reference point and the RMS voltage value of the second reference point by means of the first function to obtain an absolute value of the amplitude, and calculating a ratio of the absolute value of the amplitude to the RMS voltage value of the first reference point to obtain an amplitude decrease relative value, the absolute value of the amplitude and the amplitude decrease relative value constituting a lead positive index; wherein if the absolute value and the amplitude decrease relative value meet a predetermined condition, the lead positive index is indicated as positive.

In one embodiment, calculating the waveform change shape index according to the high-frequency QRS waveform data and by means of the second function, wherein the waveform change shape index is used for indicating the shape category to which the waveform change belongs, includes: acquiring fixed point data that the high-frequency QRS waveform data is located at a peak or a trough, the fixed point data including a time value and an RMS voltage value; the fixed point data being coordinates of a fixed point in an electrocardiogram, the fixed point being at a peak or trough position; and inputting the fixed point data into the second function, the second function outputting a waveform change shape index; wherein the waveform change shape index is used for indicating the shape category to which the waveform change belongs.

In one embodiment, the second function calculates the waveform amplitude according to the amplitude between adjacent fixed points, chooses the largest waveform amplitude calculated according to the single high-frequency QRS waveform data, filters the waveform amplitude smaller than an amplitude threshold calculated according to the largest waveform amplitude, connects the fixed-point data corresponding to the retained waveform amplitude according to the time sequence to obtain the shape function, and obtains the waveform change shape index according to the shape function.

In one embodiment, the waveform change shape index includes a first shape category, a second shape category and a third shape category; wherein the first shape category includes at least one of a U-type and an L-type, the second shape category includes at least one of a W-type, a V-type and an M-type, and the third shape category includes at least one of a flat-type and an inverted-V-type.

In one embodiment, performing analysis according to the lead positive index, the waveform change shape index, and the divided category of the positive position and by means of the second preset rule, so as to label the attention level corresponding to the high-frequency QRS waveform data, includes:
when the number of high-frequency QRS waveforms indicated as positive by the lead positive index is greater than 5, the waveform change shape index is the first shape category, and the positive location is the first category, labeling the attention level corresponding to the high-frequency QRS waveform data as a first attention level;
when the number of high-frequency QRS waveforms indicated as positive by the lead positive index is greater than 5, the waveform change shape index is the second shape category, and the positive location is the second category, labeling the attention level corresponding to the high-frequency QRS waveform data as a second attention level;
when the number of high-frequency QRS waveforms indicated as positive by the lead positive index is greater than or equal to 3 and not greater than 5, the discomfort symptom data is YES, the waveform change shape index is the first shape category, and the positive position is the first category, labeling the attention level corresponding to the high-frequency QRS waveform data as a third attention level;
when the number of high-frequency QRS waveforms indicated as positive by the lead positive index is greater than or equal to 3 and not greater than 5, the discomfort symptom data is YES, the waveform change shape index is the first shape category, and the positive position is the second category, labeling the attention level corresponding to the high-frequency QRS waveform data as a fourth attention level;
when the number of high-frequency QRS waveforms indicated as positive by the lead positive index is greater than or equal to 3 and not greater than 5, the discomfort symptom data is NO, the waveform change shape index is the second shape category, and the positive position is the first category, labeling the attention level corresponding to the high-frequency QRS waveform data as a fifth attention level;
when the number of high-frequency QRS waveforms indicated as positive by the lead positive index is greater than or equal to 3 and not greater than 5, the discomfort symptom data is NO, the waveform change shape index is the second shape category, and the positive position is the second category, labeling the attention level corresponding to the high-frequency QRS waveform data as a sixth attention level;
when the number of high-frequency QRS waveforms indicated as positive by the lead positive index is 1 or 2, the discomfort symptom data is YES, the waveform change shape index is the first shape category, and the positive position is the first category, labeling the attention level corresponding to the high-frequency QRS waveform data as a seventh attention level;
when the number of high-frequency QRS waveforms indicated as positive by the lead positive index is 1 or 2, the discomfort symptom data is YES, the waveform change shape index is the first shape category, and the positive position is the second category, labeling the attention level corresponding to the high-frequency QRS waveform data as an eighth attention level;
when the number of high-frequency QRS waveforms indicated as positive by the lead positive index is 1 or 2, the discomfort symptom data is NO, the waveform change shape index is the second shape category, and the positive position is the first category, labeling the attention level corresponding to the high-frequency QRS waveform data as a ninth attention level; and
when the number of high-frequency QRS waveforms indicated as positive by the lead positive index is 1 or 2, the discomfort symptom data is NO, the waveform change shape index is the second shape category, and the positive position is the second category, labeling the attention level corresponding to the high-frequency QRS waveform data as a tenth attention level.

In one embodiment, dividing the positive position into the first category or the second category according to the first preset rule based on the kind and quantity of the high-frequency QRS waveform data indicated as positive by the lead positive index, includes: when the high-frequency QRS waveform indicated as positive by the lead positive index is a chest lead, and the high-frequency QRS waveform data output by the chest lead is a plurality of combinations of V1, V2, V3, V4, V5 and V6, dividing the positive position into the first category according to the first preset rule; and when the high-frequency QRS waveform indicated as positive by the lead positive index is a limb lead, and the high-frequency QRS waveform data output by the limb lead is a plurality of combinations of I, II, III, aVL, aVF and aVR, dividing the positive position into the second category according to the first preset rule.

An apparatus for analyzing high-frequency QRS-complex data for non-diagnostic use, characterized in that, the apparatus includes:
a data acquisition module, used for acquiring high-frequency QRS waveform data, which is output by means of at least one electrocardiogram lead;
a lead positive index calculation module, used for calculating an amplitude decrease relative value and the absolute value of an amplitude according to the high-frequency QRS waveform data and by means of a first function, so as to form a lead positive index;
a waveform change shape index calculation module, used for calculating a waveform change shape index through a second function according to the high-frequency QRS waveform data, the waveform change shape index being used to indicate a shape category to which the waveform change belongs; and
a labeling module, used for performing analysis according to the lead positive index and the waveform change shape index and by means of a second preset rule, so as to label an attention level corresponding to the high-frequency QRS waveform data.

A computer device, including a memory and a processor, the memory storing a computer program, the processor, when executing the computer program, implementing the following steps of:
acquiring high-frequency QRS waveform data, which is output by means of at least one electrocardiogram lead;
calculating an amplitude decrease relative value and the absolute value of an amplitude according to the high-frequency QRS waveform data and by means of a first function, so as to form a lead positive index;
calculating a waveform change shape index according to the high-frequency QRS waveform data and by means of a second function, wherein the waveform change shape index is used for indicating a shape category to which a waveform change belongs; and
performing analysis according to the lead positive index and the waveform change shape index and by means of a second preset rule, so as to label an attention level corresponding to the high-frequency QRS waveform data.

A computer-readable storage medium having stored thereon a computer program, when the computer program is executed by a processor, the following steps are implemented:
acquiring high-frequency QRS waveform data, which is output by means of at least one electrocardiogram lead;
calculating an amplitude decrease relative value and the absolute value of an amplitude according to the high-frequency QRS waveform data and by means of a first function, so as to form a lead positive index;
calculating a waveform change shape index according to the high-frequency QRS waveform data and by means of a second function, wherein the waveform change shape index is used for indicating a shape category to which a waveform change belongs; and
performing analysis according to the lead positive index and the waveform change shape index and by means of a second preset rule, so as to label an attention level corresponding to the high-frequency QRS waveform data.

For the above method and apparatus for analyzing high-frequency QRS-complex data, the computer device and the storage medium, the high-frequency QRS waveform data is processed through the first and second functions to obtain the lead positive index and indicate the shape category to which a waveform change belongs. This allows for the assessment of the severity of heart problems in terms of both quantity and kind, and through the quantification of the data, the final attention level is set for doctors to refer to, which can reduce the subjectivity and time for doctors to read the electrocardiogram.

### Brief Description of Drawings

FIG. 1 is an application environment diagram of a method for analyzing high-frequency QRS-complex data in one embodiment;
FIG. 2 is a schematic diagram of a high-frequency QRS waveform in one embodiment;
FIG. 3 is a flowchart illustrating a method for analyzing high-frequency QRS-complex data in one embodiment;
FIG. 4 is a flowchart illustrating a method for analyzing high-frequency QRS-complex data in another embodiment;
FIG. 5 is a schematic diagram of a waveform shape category in one embodiment;
FIG. 6 is a structural block diagram of an apparatus for analyzing high-frequency QRS-complex data in one embodiment;
FIG. 7 is a structural block diagram of an apparatus for analyzing high-frequency QRS-complex data in one embodiment; and
FIG. 8 is a diagram of an internal structure of a computer device in one embodiment.

### Detailed Description of the Embodiments

In order to make the objects, technical solutions and advantages of the present application more clear, the present application is further described in detail below with reference to the accompanying drawings and embodiments. It should be understood that the specific embodiments described herein are merely illustrative of the present application and are not intended to limit the present application.

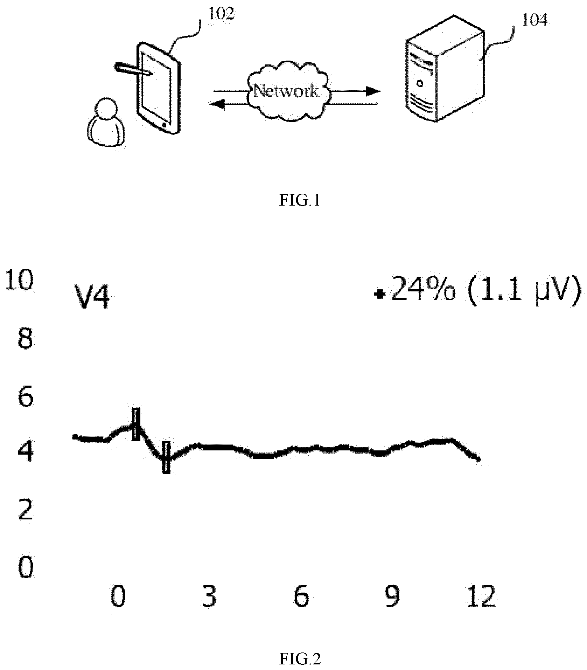

The method for analyzing high-frequency QRS-complex data provided herein can be applied to the application environment shown in FIG. 1. Wherein, the terminal 102 communicates with the server 104 over the network, the terminal 102 acquires high-frequency QRS waveform data and transmits to the server 104. The server 104 acquires high-frequency QRS waveform data output through at least one electrocardiogram lead; calculates an amplitude decrease relative value and an absolute value of an amplitude according to the high-frequency QRS waveform data and by means of a first function, so as to form a lead positive index; calculates a waveform change shape index according to the high-frequency QRS waveform data and by means of a second function, the waveform change shape index being used for indicating a shape category to which the waveform change belongs; and performs analysis according to the lead positive index and the waveform change shape index and by means of a second preset rule, so as to label an attention level corresponding to the high-frequency QRS waveform data. Wherein, the terminal 102 may be, but is not limited to, various personal computers, notebook computers, smartphones, tablet computers and portable wearable devices, the server 104 may be implemented as a stand-alone server or a server cluster of a plurality of servers.

The QRS-complex is the collection of Q, R, and S waves in the electrocardiogram. The QRS-complex reflects changes in depolarization potentials and times of left and right ventricles. The first downward wave is the Q wave, the upward wave is the R wave, and then the downward wave is the S wave. The time from the start of the QRS-complex to the end of the QRS-complex is the QRS time limit. The high-frequency refers to an electrical change with the frequency above 100 HZ, time course > 10ms, amplitude ≤ 82uv. In this application, the QRS-complex is shown by the QRS waveform output. A high-frequency QRS waveform refers to a QRS-complex that is output controlled by an electrical change of the frequency above 100 HZ, time course > 10ms, amplitude ≤ 82uv. In the high-frequency QRS waveform diagram, the abscissa is the acquisition time of the signal and the ordinate is the RMS voltage, also referred to as the root mean square voltage, as shown in FIG. 2, the kind of the high-frequency QRS waveform data is V4, the unit of the RMS voltage is uV, and the unit of time is minutes.

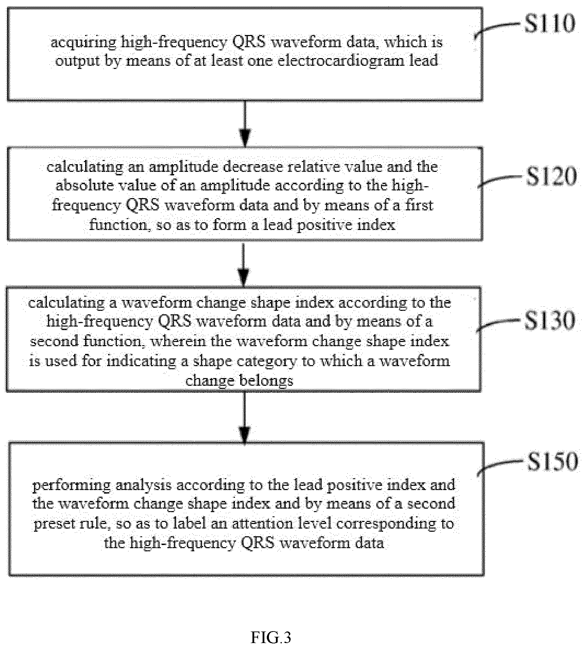

In one embodiment, as shown in FIG. 3, a method for analyzing high-frequency QRS-complex data is provided, including the following steps of:
S110, high-frequency QRS waveform data, which is output by means of at least one electrocardiogram lead, is acquired.

Wherein, in the electrocardiogram acquisition process, for example, 10 electrode pads are used to acquire, and the electrode pads are distributed on the chest and extremities of the human body to form 12 electrocardiogram leads, and 12 pieces of high-frequency QRS waveform data are outputted accordingly, and the high-frequency QRS waveform data kinds are V1, V2, V3, V4, V5, V6, I, II, III, aVL, aVF and aVR, respectively. It will be appreciated that the number of electrode pads may also be set to other numbers so as to perform electrocardiogram acquisition according to different needs.

S120, an amplitude decrease relative value and the absolute value of an amplitude are calculated according to the high-frequency QRS waveform data and by means of a first function, so as to form a lead positive index.

Wherein, the first function is used to process the high-frequency QRS waveform data, to calculate, during time period before exercise, time period in exercise and time period after exercise, the amplitude decrease relative value and the absolute value of an amplitude between the two reference points of the high-frequency QRS waveform at which the RMS voltage decreases most rapidly, the absolute value of the amplitude being obtained by difference processing the two reference points, the amplitude decrease relative value being calculated as a percentage based on the absolute value of the amplitude and the RMS voltage value at the reference point at which the RMS voltage is highest. The amplitude decrease relative value and the absolute value of the amplitude are mainly indexes of the blood flow change of the heart in human exercise.

S130, a waveform change shape index is calculated according to the high-frequency QRS waveform data and by means of a second function, wherein the waveform change shape index is used for indicating a shape category to which a waveform change belongs.

Wherein, the second function is used to process the high-frequency QRS waveform data to determine the shape of the high-frequency QRS waveform as a whole, from which the frequency and amplitude of blood flow changes in the heart can be determined. Specifically, for example, the second function can match the high-frequency QRS waveform image one by one according to the preset shape, and can determine the shape category of the high-frequency QRS waveform, e.g., the preset shape is a "U" shape, the matching is performed with the high-frequency QRS waveform image, the matching degree reaches a preset value (eighty percent), and the matching is considered to be successful, and the high-frequency QRS waveform data belongs to the U-type in the first shape category; alternatively, the second function picks out fixed points characterizing a shape change from the high-frequency QRS waveform data, determines a shape category to which the high-frequency QRS waveform data corresponds according to a change of a graph composed by the fixed points, e.g., the graph composed by the fixed points is L, the high-frequency QRS waveform data belongs to L-type in the first shape category; further alternatively, the second function chooses fixed points characterizing a shape change from the high-frequency QRS waveform data, and determines a shape category corresponding to the high-frequency QRS waveform data based on sequential changes of RMS voltages of the plurality of fixed points, e.g., if the RMS voltages of the plurality of fixed points exhibit a change of low-high-low-high-low, the high-frequency QRS waveform data belongs to M-type in the second shape category.

S150, analysis is performed according to the lead positive index and the waveform change shape index and by means of a second preset rule, so as to label an attention level corresponding to the high-frequency QRS waveform data.

Wherein, the level of cardiac health is comprehensively determined by the combined decision analysis of the lead positive index and the waveform change shape index, thereby setting different attention levels for further testing and treatment. The second preset rule determines the number of the positive high-frequency QRS waveforms according to the lead positive index, and determines the kind of abnormal data according to the waveform change shape, thereby performing integrated analysis to determine the index data of the electrocardiogram that can be read and referred to by the doctor, and the attention levels indicate different degrees of focus, and the doctor can make diagnostic and treatment reference recommendations according to the attention level and the clinical symptoms. The high-frequency QRS waveform data and the attention level can be printed together to generate a test result report to improve doctor's electrocardiogram reading efficiency.

In the above method for analyzing high-frequency QRS-complex data, the high-frequency QRS waveform data is processed by means of the first function and the second function, the lead positive index and the shape category to which the waveform change belongs are obtained, it is possible to evaluate the severity of the heart problems from the number and the kind, and through the quantification of the data, the final attention level is set for doctors to refer to, which can reduce the subjectivity and time for doctors to read the electrocardiogram.

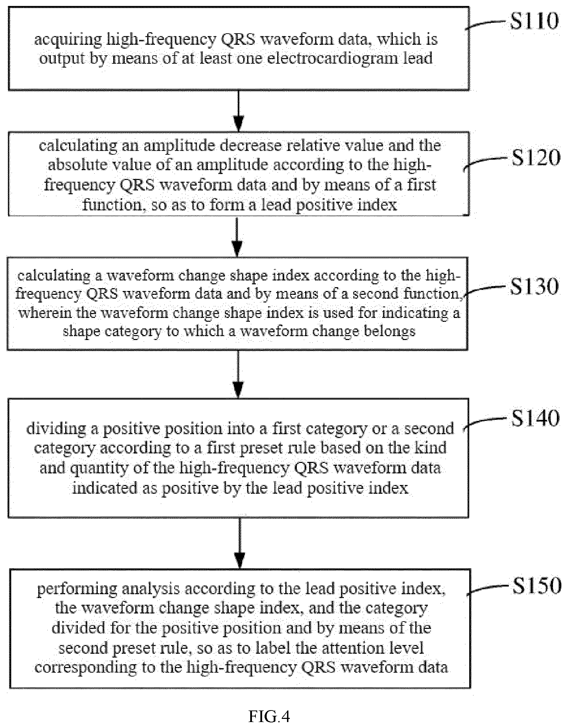

In one embodiment, as shown in FIG. 4, after the step S130, the method further includes: S140, a positive position is divided into a first category or a second category according to a first preset rule based on the kind and quantity of the high-frequency QRS waveform data indicated as positive by the lead positive index. The step S150 includes: analysis is performed according to the lead positive index, the waveform change shape index, and the divided category of the positive position and by means of the second preset rule, so as to label the attention level corresponding to the high-frequency QRS waveform data.

Wherein, the more high-frequency QRS waveform data that is positive, it is indicated that the higher the probability that there is a problem with the heart, and it is possible to determine which part of the heart (left atrium, left ventricle, right atrium, right ventricle) is likely to have a problem based on the kind of high-frequency QRS waveform data, a first preset rule is determined based on this principle, mainly for assessing where and the severity of the problem has occurred, and classifying positive locations into two categories.

Wherein, the level of cardiac health is comprehensively determined by combined decision analysis of the lead positive index, the waveform shape index and the divided category for the positive position, thereby setting different attention levels for further testing and treatment. The second preset rule can be used to determine in which locations of the heart data anomalies are likely to occur based on the combination of types of positive high-frequency QRS waveform data, and determine the kind of abnormal data according to the waveform change shape, thereby performing integrated analysis to determine the index data of the electrocardiogram that can be read and referred to by the doctor. The attention levels indicate different degrees of focus, and the doctor can make diagnostic and treatment reference recommendations according to the attention level and the clinical symptoms. The high-frequency QRS waveform data and the attention level can be printed together to generate a test result report to improve doctor's electrocardiogram reading efficiency.

In one embodiment, the step that the high-frequency QRS waveform data, which is output by means of at least one electrocardiogram lead, is acquired, comprises: high-frequency QRS waveform data for at least one sampling period, which is outputted by means of at least one electrocardiogram chest lead and/or limb lead, is acquired, the high-frequency QRS waveform data being obtained by extracting data in an interval of 150HZ-250HZ from a current signal acquired by an electrode pad applied to a human body.

Wherein, the high-frequency QRS waveform data may be from one or more electrocardiogram leads, including chest leads and/or limb leads.

In this embodiment, in acquiring the high-frequency QRS waveform data of a human body, the data is collected in three phases, including a resting phase, a exercise phase and a recovery phase, it is possible to more intuitively characterize the health of the human heart by collecting electrical signals representing changes in the blood flow of the heart during various states of the human body, so that the test result report generated later can be provided for the doctor for reference and diagnosis. The concept of exercise phase in this embodiment is not the same as the previously described exercise-time-period, the exercise-time-period include a time period before exercise, a time period in exercise, and a time period after exercise, the time period before exercise is in a resting phase, the time period in exercise includes the entire exercise phase, and the time period after exercise is in the recovery phase, the time period before exercise, the time period in exercise, and the time period after exercise are consecutive time periods.

In one embodiment, the step that the amplitude decrease relative value and the absolute value of the amplitude are calculated according to the high-frequency QRS waveform data and by means of the first function, so as to form the lead positive index, includes: exercise-time-period waveform data is intercepted from the high-frequency QRS waveform data, data at which the RMS voltage value is the largest in the exercise-time-period waveform data is chosen as a first reference point, and a point at which the RMS voltage value is the smallest after the time of the first reference point is chosen as a second reference point; and a difference between the RMS voltage value of the first reference point and the RMS voltage value of the second reference point is calculated by means of the first function to obtain an absolute value of the amplitude, and a ratio of the absolute value of the amplitude to the RMS voltage value of the first reference point is calculated to obtain an amplitude decrease relative value, the absolute value of the amplitude and the amplitude decrease relative value constitute a lead positive index; wherein if the absolute value and the amplitude decrease relative value meet a predetermined condition, the lead positive index is indicated as positive.

Wherein, the exercise-time-period waveform data includes waveform data during a time period before exercise, a time period in exercise, and a time period after exercise, e.g., the exercise-time-period waveform data includes waveform data during 100 seconds before exercise, waveform data during the exercise, and waveform data during 20 seconds after exercise.

Wherein, each of the first reference point and the second reference point includes a time value and an RMS voltage value, the time of the first reference point may be within a time period before exercise, within a time period in exercise and within a time period after exercise, and the time of the second reference point may be within a time period in exercise and within a time period after exercise, and the first reference point and the second reference point may be appropriately adjusted in accordance with the patient's age, height, weight, and the like. For example, the value of the first reference point is (0.5min, 2.5uV), the value of the second reference point is (0.15min, 2uV), the absolute value of the amplitude S = 2.5-2 = 0.5 (uV), the amplitude decrease relative value J = (0.5 ÷ 2.5) x 100% = 20%. The predetermined condition may be that the absolute value of the amplitude is greater than 0.5uV and the amplitude decrease relative value is greater than 50%. Of course, different predetermined conditions can be set in different detection environments.

In an alternative embodiment, if the absolute value and the amplitude decrease relative value meet a predetermined condition, and the lead positive index is indicated as positive, then the high-frequency QRS waveform is marked as a warning color in the electrocardiogram, for example, the high-frequency QRS waveform may be displayed as red in the electrocardiogram. Of course, the warning color is not limited to red, but may be yellow, brown, etc., as long as it can be distinguished from a normal high-frequency QRS waveform.

In one embodiment, the step that the waveform change shape index is calculated according to the high-frequency QRS waveform data and by means of the second function, wherein the waveform change shape index is used for indicating the shape category to which the waveform change belongs, includes: fixed point data that the high-frequency QRS waveform data is located at a peak or a trough is acquired, the fixed point data including a time value and an RMS voltage value; the fixed point data is coordinates of a fixed point in an electrocardiogram, the fixed point is at a peak or trough position; and the fixed point data is input into the second function, the second function outputs a waveform change shape index; wherein the waveform change shape index is used for indicating the shape category to which the waveform change belongs.

In one embodiment, the second function calculates the waveform amplitude according to the amplitude between adjacent fixed points, chooses the largest waveform amplitude calculated according to the single high-frequency QRS waveform data, filters the waveform amplitude smaller than an amplitude threshold calculated according to the largest waveform amplitude, connects the fixed-point data corresponding to the retained waveform amplitude according to the time sequence to obtain the shape function, and obtains the waveform change shape index according to the shape function.

Wherein, the waveform amplitude is obtained by subtracting the RMS voltage values between adjacent fixed points and taking absolute value of the substracting reslut; after calculating the waveform amplitude between the plurality of pairs of adjacent fixed points, the largest waveform amplitude is chosen, since the amplitude change between certain fixed points is small, the blood flow change of the heart is considered insignificant and can be left out of consideration, the filtering condition is determined by an amplitude threshold, the amplitude threshold is calculated according to the largest waveform amplitude, the amplitude threshold takes a certain proportion of the largest waveform amplitude, for example, twenty percent of the largest waveform amplitude is chosen as the amplitude threshold; of course, twenty percent is not a limitation of the present application, and other proportional values may be chosen depending on the change of the waveform. Finally, the corresponding fixed point data of the retained waveform amplitude is found, the fixed-point data is connected according to the time sequence, the shape function represents a function of the waveform after line connection, and the shape change shape index is determined according to the shape function.

In one embodiment, the waveform change shape index includes a first shape category, a second shape category and a third shape category; wherein the first shape category includes at least one of a U-type and an L-type, the second shape category includes at least one of a W-type, a V-type and an M-type, and the third shape category includes at least one of a flat-type and an inverted-V-type.

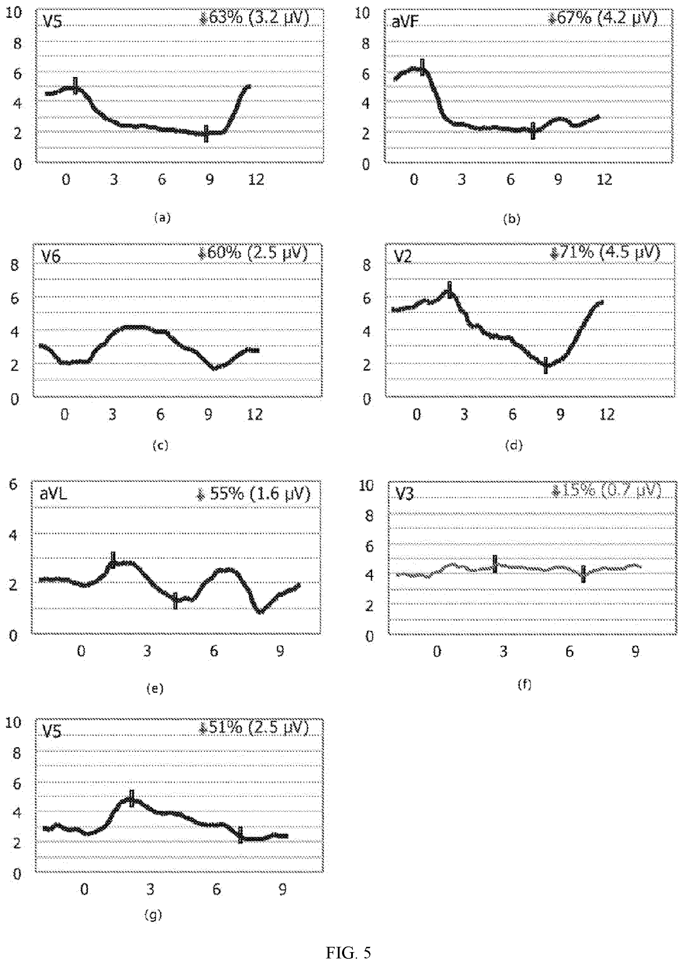

As shown in FIG. 5, the waveform change in (a) of FIG. 5 is the U-type in the first category, the waveform change in (b) of FIG. 5 is the L-type in the first category, the waveform change in (c) of FIG. 5 is the W-type in the second category, the waveform change in (d) of FIG. 5 is the V-type in the second category, the waveform change in (e) of FIG. 5 is the M-type in the second category, the waveform change in (f) of FIG. 5 is the flat-type in the third category, and the waveform change in (g) of FIG. 5 is the inverted V-type in the third category. The waveform change is a change trend of the QRS waveform as a whole.

In one embodiment, the step that analysis is performed according to the lead positive index, the waveform change shape index, and the divided category of the positive position and by means of the second preset rule, so as to label the attention level corresponding to the high-frequency QRS waveform data, includes: when the number of high-frequency QRS waveforms indicated as positive by the lead positive index is greater than 5, the waveform change shape index is the first shape category, and the positive location is the first category, the attention level corresponding to the high-frequency QRS waveform data is labeled as a first attention level;
when the number of high-frequency QRS waveforms indicated as positive by the lead positive index is greater than 5, the waveform change shape index is the second shape category, and the positive location is the second category, the attention level corresponding to the high-frequency QRS waveform data is labeled as a second attention level;
when the number of high-frequency QRS waveforms indicated as positive by the lead positive index is greater than or equal to 3 and not greater than 5, the discomfort symptom data is YES, the waveform change shape index is the first shape category, and the positive position is the first category, the attention level corresponding to the high-frequency QRS waveform data is labeled as a third attention level;
when the number of high-frequency QRS waveforms indicated as positive by the lead positive index is greater than or equal to 3 and not greater than 5, the discomfort symptom data is YES, the waveform change shape index is the first shape category, and the positive position is the second category, the attention level corresponding to the high-frequency QRS waveform data is labeled as a fourth attention level;
when the number of high-frequency QRS waveforms indicated as positive by the lead positive index is greater than or equal to 3 and not greater than 5, the discomfort symptom data is NO, the waveform change shape index is the second shape category, and the positive position is the first category, the attention level corresponding to the high-frequency QRS waveform data is labeled as a fifth attention level;
when the number of high-frequency QRS waveforms indicated as positive by the lead positive index is greater than or equal to 3 and not greater than 5, the discomfort symptom data is NO, the waveform change shape index is the second shape category, and the positive position is the second category, the attention level corresponding to the high-frequency QRS waveform data is labeled as a sixth attention level;
when the number of high-frequency QRS waveforms indicated as positive by the lead positive index is 1 or 2, the discomfort symptom data is YES, the waveform change shape index is the first shape category, and the positive position is the first category, the attention level corresponding to the high-frequency QRS waveform data is labeled as a seventh attention level;
when the number of high-frequency QRS waveforms indicated as positive by the lead positive index is 1 or 2, the discomfort symptom data is YES, the waveform change shape index is the first shape category, and the positive position is the second category, the attention level corresponding to the high-frequency QRS waveform data is labeled as an eighth attention level;
when the number of high-frequency QRS waveforms indicated as positive by the lead positive index is 1 or 2, the discomfort symptom data is NO, the waveform change shape index is the second shape category, and the positive position is the first category, the attention level corresponding to the high-frequency QRS waveform data is labeled as a ninth attention level; and
when the number of high-frequency QRS waveforms indicated as positive by the lead positive index is 1 or 2, the discomfort symptom data is NO, the waveform change shape index is the second shape category, and the positive position is the second category, the attention level corresponding to the high-frequency QRS waveform data is labeled as a tenth attention level.

Wherein, the first attention level to the tenth attention level can indicate the different degrees of heart problems by data, which can be used as a reference for doctors in the diagnosis process. For example, a first attention level may indicate that an obstructive coronary lesion is likely to occur and a clinical hospital visit is necessary; a second attention level may indicate that a non-obstructive coronary lesion is likely to occur, such as a coronary microcirculatory disorder, and a clinical hospital visit is necessary; the third and fourth attention levels may indicate that an obstructive coronary lesion is likely to occur, and further examination and determination are required; the fifth and sixth attention levels may indicate that a non-obstructive coronary lesion is likely to occur, and further examination and determination are required; the seventh and eighth attention levels require further examination; the ninth and tenth attention levels may classify this part of people into a sub-health management status, only periodic retests are required. In the absence of a high-frequency QRS waveform indicative indicated as positive by the lead positive index, and the subject has no discomfort symptoms, the heart is healthy, and only a physical examination is required.

In one embodiment, the step that the positive position is divided into the first category or the second category according to the first preset rule based on the kind and quantity of the high-frequency QRS waveform data indicated as positive by the lead positive index, includes: when the high-frequency QRS waveform indicated as positive by the lead positive index is a chest lead, and the high-frequency QRS waveform data output by the chest lead is a plurality of combinations of V1, V2, V3, V4, V5 and V6, the positive position is divided into the first category according to the first preset rule; and when the high-frequency QRS waveform indicated as positive by the lead positive index is a limb lead, and the high-frequency QRS waveform data output by the limb lead is a plurality of combinations of I, II, III, aVL, aVF and aVR, the positive position is divided into the second category according to the first preset rule.

It should be understood that while the various steps in the flowcharts of FIGS. 3 and 4 are shown in sequence as indicated by the arrows, the steps are not necessarily performed in sequence in the order indicated by the arrows. The steps are performed without a strict order limitation and the steps may be performed in other orders unless explicitly stated herein. Moreover, at least some of the steps in FIGS. 3 and 4 may include multiple steps or phases, the steps or phases are not necessarily performed at the same time, but may be performed at different times, and the order of execution of these steps or phases is not necessarily sequential. Instead, they can be performed alternately or interchangeably with other steps or at least a portion of the steps or phases of other steps.

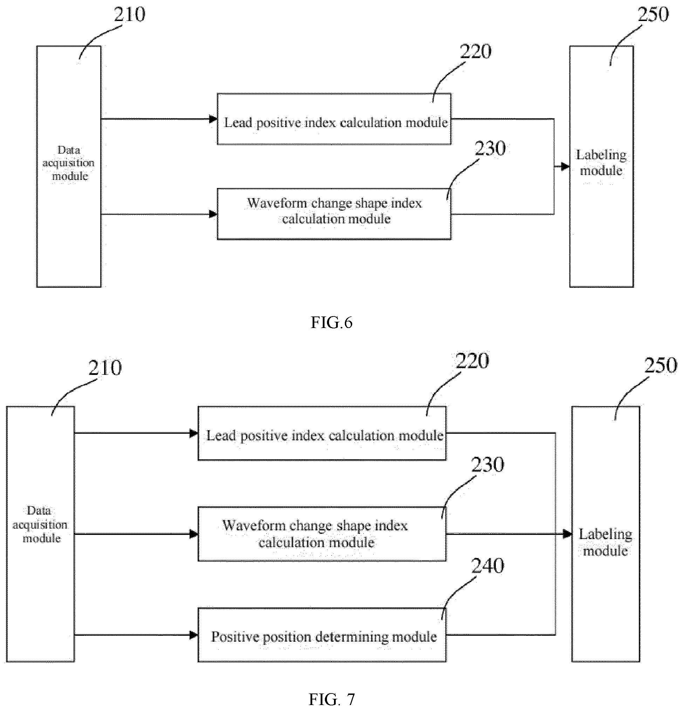

In one embodiment, as shown in FIG. 6, there is provided an apparatus for analyzing high-frequency QRS-complex data, including: a data acquisition module 210, a lead positive index calculation module 220, a waveform change shape index calculation module 230, and a labeling module 250, wherein:
The data acquisition module 210 is used for acquiring high-frequency QRS waveform data, which is output by means of at least one electrocardiogram lead.

The lead positive index calculation module 220 is used for calculating an amplitude decrease relative value and the absolute value of an amplitude according to the high-frequency QRS waveform data and by means of a first function, so as to form a lead positive index.

The waveform change shape index calculation module 230 is used for calculating a waveform change shape index through a second function according to the high-frequency QRS waveform data, the waveform change shape index being used to indicate a shape category to which the waveform change belongs.

The labeling module 250 is used for performing analysis according to the lead positive index and the waveform change shape index and by means of a second preset rule, so as to label an attention level corresponding to the high-frequency QRS waveform data.

In one embodiment, as shown in FIG. 7, the apparatus for analyzing high-frequency QRS-complex data further includes: a positive position determining module 240 used for dividing a positive position into a first category or a second category according to a first preset rule based on the kind and quantity of the high-frequency QRS waveform data indicated as positive by the lead positive index. The labeling module 250 is further used for performing analysis according to the lead positive index, the waveform change shape index, and the divided category of the positive position and by means of the second preset rule, so as to label the attention level corresponding to the high-frequency QRS waveform data.

In one embodiment, the data acquisition module 210 is further used for acquiring high-frequency QRS waveform data for at least one sampling period, which is outputted by means of at least one electrocardiogram chest lead and/or limb lead, the high-frequency QRS waveform data being obtained by extracting data in an interval of 150HZ-250HZ from a current signal acquired by an electrode pad applied to a human body. In one embodiment, the lead positive index calculation module 220 includes: a reference point choosing unit, used for intercepting exercise-time-period waveform data from the high-frequency QRS waveform data, choosing data at which the RMS voltage value is the largest in the exercise-time-period waveform data as a first reference point, and choosing a point at which the RMS voltage value is the smallest after the time of the first reference point as a second reference point; a lead positive index calculation unit, used for calculating a difference between the RMS voltage value of the first reference point and the RMS voltage value of the second reference point by means of the first function to obtain an absolute value of the amplitude, and calculating a ratio of the absolute value of the amplitude to the RMS voltage value of the first reference point to obtain an amplitude decrease relative value, the absolute value of the amplitude and the amplitude decrease relative value constituting a lead positive index; wherein if the absolute value and the amplitude decrease relative value meet a predetermined condition, the lead positive index is indicated as positive.

In one embodiment, the waveform change shape index calculation module 230 includes: a fixed point data acquisition unit, used for acquiring fixed point data that the high-frequency QRS waveform data is located at a peak or a trough, the fixed point data including a time value and an RMS voltage value; the fixed point data being coordinates of a fixed point in an electrocardiogram, the fixed point being at a peak or trough position; and a function processing unit, used for inputting the fixed point data into the second function, the second function outputting a waveform change shape index; wherein the waveform change shape index is used for indicating the shape category to which the waveform change belongs.

In one embodiment, the second function calculates the waveform amplitude according to the amplitude between adjacent fixed points, chooses the largest waveform amplitude calculated according to the single high-frequency QRS waveform data, filters the waveform amplitude smaller than an amplitude threshold calculated according to the largest waveform amplitude, connects the fixed-point data corresponding to the retained waveform amplitude according to the time sequence to obtain the shape function, and obtains the waveform change shape index according to the shape function.

In one embodiment, the waveform change shape index includes a first shape category, a second shape category and a third shape category; wherein the first shape category includes at least one of a U-type and an L-type, the second shape category includes at least one of a W-type, a V-type and an M-type, and the third shape category includes at least one of a flat-type and an inverted-V-type.

In one embodiment, the labeling module 250 includes:
a first labeling unit, used for, when the number of high-frequency QRS waveforms indicated as positive by the lead positive index is greater than 5, the waveform change shape index is the first shape category, and the positive location is the first category, labeling the attention level corresponding to the high-frequency QRS waveform data as a first attention level;
a second labeling unit, used for, when the number of high-frequency QRS waveforms indicated as positive by the lead positive index is greater than 5, the waveform change shape index is the second shape category, and the positive location is the second category, labeling the attention level corresponding to the high-frequency QRS waveform data as a second attention level;
a third labeling unit, used for, when the number of high-frequency QRS waveforms indicated as positive by the lead positive index is greater than or equal to 3 and not greater than 5, the discomfort symptom data is YES, the waveform change shape index is the first shape category, and the positive position is the first category, labeling the attention level corresponding to the high-frequency QRS waveform data as a third attention level;
a fourth labeling unit, used for, when the number of high-frequency QRS waveforms indicated as positive by the lead positive index is greater than or equal to 3 and not greater than 5, the discomfort symptom data is YES, the waveform change shape index is the first shape category, and the positive position is the second category, labeling the attention level corresponding to the high-frequency QRS waveform data as a fourth attention level;
a fifth labeling unit, used for, when the number of high-frequency QRS waveforms indicated as positive by the lead positive index is greater than or equal to 3 and not greater than 5, the discomfort symptom data is NO, the waveform change shape index is the second shape category, and the positive position is the first category, labeling the attention level corresponding to the high-frequency QRS waveform data as a fifth attention level;
a sixth labeling unit, used for, when the number of high-frequency QRS waveforms indicated as positive by the lead positive index is greater than or equal to 3 and not greater than 5, the discomfort symptom data is NO, the waveform change shape index is the second shape category, and the positive position is the second category, labeling the attention level corresponding to the high-frequency QRS waveform data as a sixth attention level;
a seventh labeling unit, used for, when the number of high-frequency QRS waveforms indicated as positive by the lead positive index is 1 or 2, the discomfort symptom data is YES, the waveform change shape index is the first shape category, and the positive position is the first category, labeling the attention level corresponding to the high-frequency QRS waveform data as a seventh attention level;
an eighth labeling unit, used for, when the number of high-frequency QRS waveforms indicated as positive by the lead positive index is 1 or 2, the discomfort symptom data is YES, the waveform change shape index is the first shape category, and the positive position is the second category, labeling the attention level corresponding to the high-frequency QRS waveform data as an eighth attention level;
a ninth labeling unit, used for, when the number of high-frequency QRS waveforms indicated as positive by the lead positive index is 1 or 2, the discomfort symptom data is NO, the waveform change shape index is the second shape category, and the positive position is the first category, labeling the attention level corresponding to the high-frequency QRS waveform data as a ninth attention level; and
a tenth labeling unit, used for, when the number of high-frequency QRS waveforms indicated as positive by the lead positive index is 1 or 2, the discomfort symptom data is NO, the waveform change shape index is the second shape category, and the positive position is the second category, labeling the attention level corresponding to the high-frequency QRS waveform data as a tenth attention level.

In one embodiment, the positive position determining module 240 includes: a first dividing unit, used for, when the high-frequency QRS waveform indicated as positive by the lead positive index is a chest lead, and the high-frequency QRS waveform data output by the chest lead is a plurality of combinations of V1, V2, V3, V4, V5 and V6, dividing the positive position into the first category according to the first preset rule; and a second dividing unit, used for, when the high-frequency QRS waveform indicated as positive by the lead positive index is a limb lead, and the high-frequency QRS waveform data output by the limb lead is a plurality of combinations of I, II, III, aVL, aVF and aVR, dividing the positive position into the second category according to the first preset rule.

The specific limitation for the apparatus for analyzing high-frequency QRS-complex data can refer to the limitation for the method for analyzing high-frequency QRS-complex data described above, which will not be repeated here. The various modules in the apparatus for analyzing high-frequency QRS-complex data described above may be implemented in whole or in part by software, hardware, and combinations thereof. Each module can be embedded in or independent of the processor in the computer device in the form of hardware, and can also be stored in the memory in the computer device in the form of software, so as to facilitate the processor to call and execute the operations corresponding to each module.

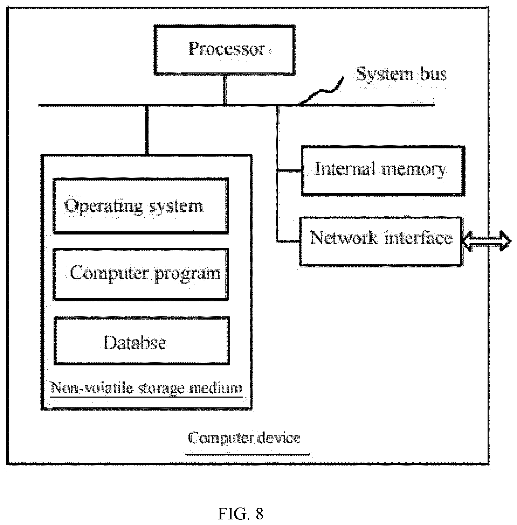

In one embodiment, there is provided a computer device, which may be a server, whose internal structure diagram may be as shown in FIG. 8. The computer device includes a processor, a memory, and a network interface connected by a system bus. Wherein, the processor of the computer device is configured to provide computing and control capabilities. The memory of the computer device includes a non-volatile storage medium, and an internal memory. The non-volatile storage medium stores an operating system, a computer program and a database. The internal memory provides an environment for an operating system and the running of the computer program in the non-volatile storage medium. The database of the computer device is configured to store high-frequency QRS waveform data. The network interface of the computer device is configured to communicate with an external terminal over a network connection. The computer program, when executed by the processor, implements the method for analyzing high-frequency QRS-complex data.

Those skilled in the art can appreciate that the structure shown in FIG. 8 is merely a block diagram of a portion of the structure relevant to the present application, and does not constitute a limitation on the computer device to which the present application is applied, and a particular computer device may include more or fewer components than shown in the figure, or combine some components, or have a different arrangement of components.

In one embodiment, there is also provided a computer device including a memory having a computer program stored therein and a processor which, when executes the computer program, implements the steps of the above method embodiments.

In one embodiment, there is provided a computer-readable storage medium having stored thereon a computer program which, when executed by a processor, implements the steps of the above method embodiments.

Those of ordinary skill in the art can understand that all or part of the flows in the above embodiment methods can be performed by instructing related hardware through a computer program stored in a non-volatile computer readable storage medium, and the computer program, when executed, may include the flows of the embodiments of the above-described methods. Wherein, any reference to the memory, storage, database, or other media used in various embodiments provided herein can include at least one of non-volatile and volatile memories. The non-volatile memory may include a Read-Only Memory (ROM), a magnetic tape, a floppy disk, a flash memory, or an optical memory. The volatile memory can include a Random Access Memory (RAM) or an external cache memory. By way of illustration and not limitation, the RAM may take many forms such as a Static Random Access Memory (SRAM) or a Dynamic Random Access Memory (DRAM), etc.

The technical features of the above embodiments may be combined arbitrarily, and all possible combinations of the technical features of the above embodiments have not been described in order to make the description concise. However, as long as there is no contradiction in the combinations of these technical features, they should be considered as the scope described in this specification.

The above-described embodiments are merely illustrative of several implementation modes of the present application and the description thereof is more specific and detailed but cannot therefore be construed as limiting the scope of the invention. It should be noted that a number of modifications and improvements may be made by those of ordinary skill in the art without departing from the concept of the present application, which fall within the scope of protection of the present application. Therefore, the scope of protection of the patent application shall be subject to the attached claims.

## Claims

1. An apparatus for analyzing high-frequency QRS-complex data, the apparatus comprises:
a data acquisition module (210), configured to acquire high-frequency QRS waveform data, which is output by means of at least one electrocardiogram lead, wherein the high-frequency QRS waveform data comprises exercise-time-period waveform data which includes waveform data during a time period before exercise, a time period in exercise, and a time period after exercise;
a lead positive index calculation module (220), configured to calculate an amplitude decrease relative value and the absolute value of an amplitude according to the high-frequency QRS waveform data and by means of a first function, so as to form a lead positive index;
a waveform change shape index calculation module (230), **characterized in that**, the waveform change shape index calculation module is configured to calculate a waveform change shape index through a second function according to the high-frequency QRS waveform data, the waveform change shape index configured to indicate a shape category to which the waveform change belongs, wherein the waveform change shape index comprises a first shape category, a second shape category and a third shape category; wherein the first shape category comprises at least one of a U-type and an L-type, the second shape category comprises at least one of a W-type, a V-type and an M-type, and the third shape category comprises at least one of a flat-type and an inverted-V-type;
a labeling module (250), configured to perform analysis according to the lead positive index and the waveform change shape index and by means of a second preset rule, so as to label an attention level corresponding to the high-frequency QRS waveform data; and
a positive position determining module (240), configured to divide a positive position into a first category or a second category according to a first preset rule based on the kind and quantity of the high-frequency QRS waveform data indicated as positive by the lead positive index;
wherein the labeling module is configured to determine the attention level based on:
(i) the number of high-frequency QRS waveforms indicated as positive by the lead positive index,
(ii) the waveform change shape index, and
(iii) the divided category of the positive position,
so as to determine the attention level corresponding to the high-frequency QRS waveform data;
wherein the second preset rule defines different attention levels for different analysis results obtained from said combined analysis,
wherein calculating the waveform change shape index according to the high-frequency QRS waveform data and by means of the second function, comprises:
acquiring fixed point data that the high-frequency QRS waveform data is located at a peak or a trough, the fixed point data comprising a time value and an RMS voltage value; the fixed point data being coordinates of a fixed point in an electrocardiogram, the fixed point being at a peak or trough position; and
inputting the fixed point data into the second function, the second function outputting a waveform change shape index; and
wherein the second function calculates the waveform amplitude according to the amplitude between adjacent fixed points, chooses the largest waveform amplitude calculated according to the single high-frequency QRS waveform data, filters the waveform amplitude smaller than an amplitude threshold calculated according to the largest waveform amplitude, connects the fixed-point data corresponding to the retained waveform amplitude according to the time sequence to obtain the shape function, and obtains the waveform change shape index according to the shape function.

2. The apparatus according to claim 1, wherein the data acquisition module (210) is further used for acquiring the high-frequency QRS waveform data for at least one sampling period, which is outputted by means of at least one electrocardiogram chest lead and/or limb lead, the high-frequency QRS waveform data being obtained by extracting data in an interval of 150HZ-250HZ from a current signal acquired by an electrode pad applied to a human body.

3. The apparatus according to claim 1, wherein the lead positive index calculation module (220) comprises:
a reference point choosing unit, used for intercepting exercise-time-period waveform data from the high-frequency QRS waveform data, choosing data at which the RMS voltage value is the largest in the exercise-time-period waveform data as a first reference point, and choosing a point at which the RMS voltage value is the smallest after the time of the first reference point as a second reference point; and
a lead positive index calculation unit, used for calculating a difference between the RMS voltage value of the first reference point and the RMS voltage value of the second reference point by means of the first function to obtain an absolute value of the amplitude, and calculating a ratio of the absolute value of the amplitude to the RMS voltage value of the first reference point to obtain an amplitude decrease relative value, the absolute value of the amplitude and the amplitude decrease relative value constituting a lead positive index; wherein if the absolute value and the amplitude decrease relative value meet a predetermined condition, the lead positive index is indicated as positive.

4. The apparatus according to claim 1, wherein the labeling module (250) comprises:
a first labeling unit, used for, when the number of high-frequency QRS waveforms indicated as positive by the lead positive index is greater than 5, the waveform change shape index is the first shape category, and the positive location is the first category, labeling the attention level corresponding to the high-frequency QRS waveform data as a first attention level;
a second labeling unit, used for, when the number of high-frequency QRS waveforms indicated as positive by the lead positive index is greater than 5, the waveform change shape index is the second shape category, and the positive location is the second category, labeling the attention level corresponding to the high-frequency QRS waveform data as a second attention level;
a third labeling unit, used for, when the number of high-frequency QRS waveforms indicated as positive by the lead positive index is greater than or equal to 3 and not greater than 5, the discomfort symptom data is YES, the waveform change shape index is the first shape category, and the positive position is the first category, labeling the attention level corresponding to the high-frequency QRS waveform data as a third attention level;
a fourth labeling unit, used for, when the number of high-frequency QRS waveforms indicated as positive by the lead positive index is greater than or equal to 3 and not greater than 5, the discomfort symptom data is YES, the waveform change shape index is the first shape category, and the positive position is the second category, labeling the attention level corresponding to the high-frequency QRS waveform data as a fourth attention level;
a fifth labeling unit, used for, when the number of high-frequency QRS waveforms indicated as positive by the lead positive index is greater than or equal to 3 and not greater than 5, the discomfort symptom data is NO, the waveform change shape index is the second shape category, and the positive position is the first category, labeling the attention level corresponding to the high-frequency QRS waveform data as a fifth attention level;
a sixth labeling unit, used for, when the number of high-frequency QRS waveforms indicated as positive by the lead positive index is greater than or equal to 3 and not greater than 5, the discomfort symptom data is NO, the waveform change shape index is the second shape category, and the positive position is the second category, labeling the attention level corresponding to the high-frequency QRS waveform data as a sixth attention level;
a seventh labeling unit, used for, when the number of high-frequency QRS waveforms indicated as positive by the lead positive index is 1 or 2, the discomfort symptom data is YES, the waveform change shape index is the first shape category, and the positive position is the first category, labeling the attention level corresponding to the high-frequency QRS waveform data as a seventh attention level;
an eighth labeling unit, used for, when the number of high-frequency QRS waveforms indicated as positive by the lead positive index is 1 or 2, the discomfort symptom data is YES, the waveform change shape index is the first shape category, and the positive position is the second category, labeling the attention level corresponding to the high-frequency QRS waveform data as an eighth attention level;
a ninth labeling unit, used for, when the number of high-frequency QRS waveforms indicated as positive by the lead positive index is 1 or 2, the discomfort symptom data is NO, the waveform change shape index is the second shape category, and the positive position is the first category, labeling the attention level corresponding to the high-frequency QRS waveform data as a ninth attention level; and
a tenth labeling unit, used for, when the number of high-frequency QRS waveforms indicated as positive by the lead positive index is 1 or 2, the discomfort symptom data is NO, the waveform change shape index is the second shape category, and the positive position is the second category, labeling the attention level corresponding to the high-frequency QRS waveform data as a tenth attention level.

5. The apparatus according to claim 1, wherein the positive position determining module (240) comprises:
a first dividing unit, used for, when the high-frequency QRS waveform indicated as positive by the lead positive index is a chest lead, and the high-frequency QRS waveform data output by the chest lead is a plurality of combinations of V1, V2, V3, V4, V5 and V6, dividing the positive position into the first category according to the first preset rule; and
a second dividing unit, used for, when the high-frequency QRS waveform indicated as positive by the lead positive index is a limb lead, and the high-frequency QRS waveform data output by the limb lead is a plurality of combinations of I, II, III, aVL, aVF and aVR, dividing the positive position into the second category according to the first preset rule.

6. A computer device, comprising a memory and a processor, the memory storing a computer program, wherein the processor, when executing the computer program, implements a method for analyzing high-frequency QRS-complex data, which comprises:
acquiring high-frequency QRS waveform data from a data acquisition module (210), which is output by means of at least one electrocardiogram lead, wherein the high-frequency QRS waveform data comprises exercise-time-period waveform data which includes waveform data during a time period before exercise, a time period in exercise, and a time period after exercise;
calculating an amplitude decrease relative value and the absolute value of an amplitude according to the high-frequency QRS waveform data and by means of a first function, so as to form a lead positive index;
**characterized in that** a waveform change shape index is calculated through a second function according to the high-frequency QRS waveform data, wherein the waveform change shape index is used for indicating a shape category to which the waveform change belongs, wherein the waveform change shape index comprises a first shape category, a second shape category and a third shape category; wherein the first shape category comprises at least one of a U-type and an L-type, the second shape category comprises at least one of a W-type, a V-type and an M-type, and the third shape category comprises at least one of a flat-type and an inverted-V-type;
performing analysis according to the lead positive index and the waveform change shape index and by means of a second preset rule, so as to label an attention level corresponding to the high-frequency QRS waveform data; and
dividing a positive position into a first category or a second category according to a first preset rule based on the kind and quantity of the high-frequency QRS waveform data indicated as positive by the lead positive index;
determining the attention level based on:
(i) the number of high-frequency QRS waveforms indicated as positive by the lead positive index,
(ii) the waveform change shape index, and
(iii) the divided category of the positive position,
so as to determine the attention level corresponding to the high-frequency QRS waveform data;
wherein the second preset rule defines different attention levels for different analysis results obtained from said combined analysis,
wherein calculating the waveform change shape index according to the high-frequency QRS waveform data and by means of the second function , comprises:
acquiring fixed point data that the high-frequency QRS waveform data is located at a peak or a trough, the fixed point data comprising a time value and an RMS voltage value; the fixed point data being coordinates of a fixed point in an electrocardiogram, the fixed point being at a peak or trough position; and
inputting the fixed point data into the second function, the second function outputting a waveform change shape index; and
wherein the second function calculates the waveform amplitude according to the amplitude between adjacent fixed points, chooses the largest waveform amplitude calculated according to the single high-frequency QRS waveform data, filters the waveform amplitude smaller than an amplitude threshold calculated according to the largest waveform amplitude, connects the fixed-point data corresponding to the retained waveform amplitude according to the time sequence to obtain the shape function, and obtains the waveform change shape index according to the shape function.

7. The computer device according to claim 6, wherein acquiring high-frequency QRS waveform data comprises:
acquiring the high-frequency QRS waveform data for at least one sampling period, which is outputted by means of at least one electrocardiogram chest lead and/or limb lead, the high-frequency QRS waveform data being obtained by extracting data in an interval of 150HZ-250HZ from a current signal acquired by an electrode pad applied to a human body.

8. The computer device according to claim 6, wherein calculating an amplitude decrease relative value and the absolute value of an amplitude according to the high-frequency QRS waveform data and by means of a first function, so as to form a lead positive index comprises:
intercepting exercise-time-period waveform data from the high-frequency QRS waveform data, choosing data at which the RMS voltage value is the largest in the exercise-time-period waveform data as a first reference point, and choosing a point at which the RMS voltage value is the smallest after the time of the first reference point as a second reference point; and
calculating a difference between the RMS voltage value of the first reference point and the RMS voltage value of the second reference point by means of the first function to obtain an absolute value of the amplitude, and calculating a ratio of the absolute value of the amplitude to the RMS voltage value of the first reference point to obtain an amplitude decrease relative value, the absolute value of the amplitude and the amplitude decrease relative value constituting a lead positive index; wherein if the absolute value and the amplitude decrease relative value meet a predetermined condition, the lead positive index is indicated as positive.

9. A computer-readable storage medium having stored thereon a computer program, wherein when the computer program is executed by a processor, causes the processor to perform a method for analyzing high-frequency QRS-complex data, which comprises:
acquiring high-frequency QRS waveform data from a data acquisition module (210),, which is output by means of at least one electrocardiogram lead, wherein the high-frequency QRS waveform data comprises exercise-time-period waveform data which includes waveform data during a time period before exercise, a time period in exercise, and a time period after exercise;
calculating an amplitude decrease relative value and the absolute value of an amplitude according to the high-frequency QRS waveform data and by means of a first function, so as to form a lead positive index;
**characterized in that** a waveform change shape index is calculated through a second function according to the high-frequency QRS waveform data, wherein the waveform change shape index is used for indicating a shape category to which the waveform change belongs, wherein the waveform change shape index comprises a first shape category, a second shape category and a third shape category; wherein the first shape category comprises at least one of a U-type and an L-type, the second shape category comprises at least one of a W-type, a V-type and an M-type, and the third shape category comprises at least one of a flat-type and an inverted-V-type;
performing analysis according to the lead positive index and the waveform change shape index and by means of a second preset rule, so as to label an attention level corresponding to the high-frequency QRS waveform data; and
dividing a positive position into a first category or a second category according to a first preset rule based on the kind and quantity of the high-frequency QRS waveform data indicated as positive by the lead positive index; determining the attention level based on:
(i) the number of high-frequency QRS waveforms indicated as positive by the lead positive index,
(ii) the waveform change shape index, and
(iii) the divided category of the positive position,
so as to determine the attention level corresponding to the high-frequency QRS waveform data;
wherein the second preset rule defines different attention levels for different analysis results obtained from said combined analysis,
wherein calculating the waveform change shape index according to the high-frequency QRS waveform data and by means of the second function, comprises:
acquiring fixed point data that the high-frequency QRS waveform data is located at a peak or a trough, the fixed point data comprising a time value and an RMS voltage value; the fixed point data being coordinates of a fixed point in an electrocardiogram, the fixed point being at a peak or trough position; and
inputting the fixed point data into the second function, the second function outputting a waveform change shape index; and
wherein the second function calculates the waveform amplitude according to the amplitude between adjacent fixed points, chooses the largest waveform amplitude calculated according to the single high-frequency QRS waveform data, filters the waveform amplitude smaller than an amplitude threshold calculated according to the largest waveform amplitude, connects the fixed-point data corresponding to the retained waveform amplitude according to the time sequence to obtain the shape function, and obtains the waveform change shape index according to the shape function.

10. The computer-readable storage medium according to claim 9, wherein acquiring high-frequency QRS waveform data comprises:
acquiring the high-frequency QRS waveform data for at least one sampling period, which is outputted by means of at least one electrocardiogram chest lead and/or limb lead, the high-frequency QRS waveform data being obtained by extracting data in an interval of 150HZ-250HZ from a current signal acquired by an electrode pad applied to a human body.

11. The computer-readable storage medium according to claim 9, wherein calculating an amplitude decrease relative value and the absolute value of an amplitude according to the high-frequency QRS waveform data and by means of a first function, so as to form a lead positive index comprises:
intercepting exercise-time-period waveform data from the high-frequency QRS waveform data, choosing data at which the RMS voltage value is the largest in the exercise-time-period waveform data as a first reference point, and choosing a point at which the RMS voltage value is the smallest after the time of the first reference point as a second reference point; and
calculating a difference between the RMS voltage value of the first reference point and the RMS voltage value of the second reference point by means of the first function to obtain an absolute value of the amplitude, and calculating a ratio of the absolute value of the amplitude to the RMS voltage value of the first reference point to obtain an amplitude decrease relative value, the absolute value of the amplitude and the amplitude decrease relative value constituting a lead positive index; wherein if the absolute value and the amplitude decrease relative value meet a predetermined condition, the lead positive index is indicated as positive.

## Patentansprüche

1. Vorrichtung zur Analyse von Hochfrequenz-QRS-Komplex-Daten, wobei die Vorrichtung umfasst:
ein Datenerfassungsmodul (210), das dazu eingerichtet ist, Hochfrequenz-QRS-Wellenformdaten zu erfassen, die mittels mindestens einer Elektrokardiogramm-Ableitung ausgegeben werden, wobei die Hochfrequenz-QRS-Wellenformdaten Belastungszeitraum-Wellenformdaten umfassen, die Wellenformdaten während eines Zeitraums vor der Belastung, eines Zeitraums während der Belastung und eines Zeitraums nach der Belastung beinhalten;
ein Modul (220) zur Berechnung eines ableitungspositiven Index, das dazu eingerichtet ist, einen relativen Amplitudenabfallwert und den Absolutwert einer Amplitude anhand der Hochfrequenz-QRS-Wellenformdaten und mittels einer ersten Funktion zu berechnen, um einen ableitungspositiven Index zu bilden;
ein Modul (230) zur Berechnung eines Wellenformänderungs-Formindex, **dadurch gekennzeichnet, dass** das Modul zur Berechnung eines Wellenformänderungs-Formindex dazu eingerichtet ist, über eine zweite Funktion anhand der Hochfrequenz-QRS-Wellenformdaten einen Wellenformänderungs-Formindex zu berechnen, wobei der Wellenformänderungs-Formindex dazu eingerichtet ist, eine Formkategorie anzugeben, zu der die Wellenformänderung gehört, wobei der Wellenformänderungs-Formindex eine erste Formkategorie, eine zweite Formkategorie und eine dritte Formkategorie umfasst; wobei die erste Formkategorie mindestens eines von einem U-Typ und einem L-Typ umfasst, die zweite Formkategorie mindestens eines von einem W-Typ, einem V-Typ und einem M-Typ umfasst und die dritte Formkategorie mindestens eines von einem flachen Typ und einem invertierten V-Typ umfasst;
ein Kennzeichnungsmodul (250), das dazu eingerichtet ist, eine Analyse anhand des ableitungspositiven Index und des Wellenformänderungs-Formindex sowie mittels einer zweiten voreingestellten Regel durchzuführen, um eine den Hochfrequenz-QRS-Wellenformdaten entsprechende Aufmerksamkeitsstufe zu kennzeichnen; und
ein Modul (240) zur Bestimmung einer positiven Position, das dazu eingerichtet ist, eine positive Position gemäß einer ersten voreingestellten Regel basierend auf der Art und Menge der durch den ableitungspositiven Index als positiv angegebenen Hochfrequenz-QRS-Wellenformdaten in eine erste Kategorie oder eine zweite Kategorie zu unterteilen;
wobei das Kennzeichnungsmodul dazu eingerichtet ist, die Aufmerksamkeitsstufe basierend auf:
(i) der Anzahl von durch den ableitungspositiven Index als positiv angegebenen Hochfrequenz-QRS-Wellenformen,
(ii) dem Wellenformänderungs-Formindex und
(iii) der unterteilten Kategorie der positiven Position,
zu bestimmen, um die den Hochfrequenz-QRS-Wellenformdaten entsprechende Aufmerksamkeitsstufe zu bestimmen;
wobei die zweite voreingestellte Regel unterschiedliche Aufmerksamkeitsstufen für unterschiedliche Analyseergebnisse definiert, die aus der kombinierten Analyse erhalten werden,
wobei das Berechnen des Wellenformänderungs-Formindex anhand der Hochfrequenz-QRS-Wellenformdaten und mittels der zweiten Funktion umfasst:
Erfassen von Festpunktdaten, an denen sich die Hochfrequenz-QRS-Wellenformdaten an einem Peak oder einem Tal befinden, wobei die Festpunktdaten einen Zeitwert und einen Effektivspannungswert (RMS) umfassen; wobei die Festpunktdaten Koordinaten eines Festpunkts in einem Elektrokardiogramm sind, wobei der Festpunkt an einer Peak- oder Talposition liegt; und
Eingeben der Festpunktdaten in die zweite Funktion, wobei die zweite Funktion einen Wellenformänderungs-Formindex ausgibt; und
wobei die zweite Funktion die Wellenformamplitude anhand der Amplitude zwischen benachbarten Festpunkten berechnet, die größte anhand der einzelnen Hochfrequenz-QRS-Wellenformdaten berechnete Wellenformamplitude auswählt, die Wellenformamplitude filtert, die kleiner als ein anhand der größten Wellenformamplitude berechneter Amplitudenschwellenwert ist, die der beibehaltenen Wellenformamplitude entsprechenden Festpunktdaten gemäß der zeitlichen Abfolge verbindet, um die Formfunktion zu erhalten, und den Wellenformänderungs-Formindex anhand der Formfunktion erhält.

2. Vorrichtung nach Anspruch 1, wobei das Datenerfassungsmodul (210) ferner dazu dient, die Hochfrequenz-QRS-Wellenformdaten für mindestens eine Abtastperiode zu erfassen, die mittels mindestens einer Elektrokardiogramm-Brustwandableitung und/oder -Extremitätenableitung ausgegeben werden, wobei die Hochfrequenz-QRS-Wellenformdaten durch Extrahieren von Daten in einem Intervall von 150 Hz bis 250 Hz aus einem Stromsignal erhalten werden, das durch ein an einem menschlichen Körper angebrachtes Elektrodenpad erfasst wird.

3. Vorrichtung nach Anspruch 1, wobei das Modul (220) zur Berechnung eines ableitungspositiven Index umfasst:
eine Referenzpunkt-Auswahleinheit, die dazu dient, Belastungszeitraum-Wellenformdaten aus den Hochfrequenz-QRS-Wellenformdaten abzufangen, Daten, bei denen der Effektivspannungswert (RMS) in den Belastungszeitraum-Wellenformdaten am größten ist, als einen ersten Referenzpunkt auszuwählen und einen Punkt, an dem der Effektivspannungswert (RMS) nach dem Zeitpunkt des ersten Referenzpunkts am kleinsten ist, als einen zweiten Referenzpunkt auszuwählen; und
eine Einheit zur Berechnung eines ableitungspositiven Index, die dazu dient, eine Differenz zwischen dem Effektivspannungswert (RMS) des ersten Referenzpunkts und dem Effektivspannungswert (RMS) des zweiten Referenzpunkts mittels der ersten Funktion zu berechnen, um einen Absolutwert der Amplitude zu erhalten, und ein Verhältnis des Absolutwerts der Amplitude zum Effektivspannungswert (RMS) des ersten Referenzpunkts zu berechnen, um einen relativen Amplitudenabfallwert zu erhalten, wobei der Absolutwert der Amplitude und der relative Amplitudenabfallwert einen ableitungspositiven Index bilden; wobei, wenn der Absolutwert und der relative Amplitudenabfallwert eine vorbestimmte Bedingung erfüllen, der ableitungspositive Index als positiv angegeben wird.

4. Vorrichtung nach Anspruch 1, wobei das Kennzeichnungsmodul (250) umfasst:
eine erste Kennzeichnungseinheit, die dazu dient, wenn die Anzahl von durch den ableitungspositiven Index als positiv angegebenen Hochfrequenz-QRS-Wellenformen größer als 5 ist, der Wellenformänderungs-Formindex die erste Formkategorie ist und die positive Position die erste Kategorie ist, die den Hochfrequenz-QRS-Wellenformdaten entsprechende Aufmerksamkeitsstufe als eine erste Aufmerksamkeitsstufe zu kennzeichnen;
eine zweite Kennzeichnungseinheit, die dazu dient, wenn die Anzahl von durch den ableitungspositiven Index als positiv angegebenen Hochfrequenz-QRS-Wellenformen größer als 5 ist, der Wellenformänderungs-Formindex die zweite Formkategorie ist und die positive Position die zweite Kategorie ist, die den Hochfrequenz-QRS-Wellenformdaten entsprechende Aufmerksamkeitsstufe als eine zweite Aufmerksamkeitsstufe zu kennzeichnen;
eine dritte Kennzeichnungseinheit, die dazu dient, wenn die Anzahl von durch den ableitungspositiven Index als positiv angegebenen Hochfrequenz-QRS-Wellenformen größer oder gleich 3 und nicht größer als 5 ist, die Beschwerdesymptomdaten JA lauten, der Wellenformänderungs-Formindex die erste Formkategorie ist und die positive Position die erste Kategorie ist, die den Hochfrequenz-QRS-Wellenformdaten entsprechende Aufmerksamkeitsstufe als eine dritte Aufmerksamkeitsstufe zu kennzeichnen;
eine vierte Kennzeichnungseinheit, die dazu dient, wenn die Anzahl von durch den ableitungspositiven Index als positiv angegebenen Hochfrequenz-QRS-Wellenformen größer oder gleich 3 und nicht größer als 5 ist, die Beschwerdesymptomdaten JA lauten, der Wellenformänderungs-Formindex die erste Formkategorie ist und die positive Position die zweite Kategorie ist, die den Hochfrequenz-QRS-Wellenformdaten entsprechende Aufmerksamkeitsstufe als eine vierte Aufmerksamkeitsstufe zu kennzeichnen;
eine fünfte Kennzeichnungseinheit, die dazu dient, wenn die Anzahl von durch den ableitungspositiven Index als positiv angegebenen Hochfrequenz-QRS-Wellenformen größer oder gleich 3 und nicht größer als 5 ist, die Beschwerdesymptomdaten NEIN lauten, der Wellenformänderungs-Formindex die zweite Formkategorie ist und die positive Position die erste Kategorie ist, die den Hochfrequenz-QRS-Wellenformdaten entsprechende Aufmerksamkeitsstufe als eine fünfte Aufmerksamkeitsstufe zu kennzeichnen;
eine sechste Kennzeichnungseinheit, die dazu dient, wenn die Anzahl von durch den ableitungspositiven Index als positiv angegebenen Hochfrequenz-QRS-Wellenformen größer oder gleich 3 und nicht größer als 5 ist, die Beschwerdesymptomdaten NEIN lauten, der Wellenformänderungs-Formindex die zweite Formkategorie ist und die positive Position die zweite Kategorie ist, die den Hochfrequenz-QRS-Wellenformdaten entsprechende Aufmerksamkeitsstufe als eine sechste Aufmerksamkeitsstufe zu kennzeichnen;
eine siebte Kennzeichnungseinheit, die dazu dient, wenn die Anzahl von durch den ableitungspositiven Index als positiv angegebenen Hochfrequenz-QRS-Wellenformen 1 oder 2 ist, die Beschwerdesymptomdaten JA lauten, der Wellenformänderungs-Formindex die erste Formkategorie ist und die positive Position die erste Kategorie ist, die den Hochfrequenz-QRS-Wellenformdaten entsprechende Aufmerksamkeitsstufe als eine siebte Aufmerksamkeitsstufe zu kennzeichnen;
eine achte Kennzeichnungseinheit, die dazu dient, wenn die Anzahl von durch den ableitungspositiven Index als positiv angegebenen Hochfrequenz-QRS-Wellenformen 1 oder 2 ist, die Beschwerdesymptomdaten JA lauten, der Wellenformänderungs-Formindex die erste Formkategorie ist und die positive Position die zweite Kategorie ist, die den Hochfrequenz-QRS-Wellenformdaten entsprechende Aufmerksamkeitsstufe als eine achte Aufmerksamkeitsstufe zu kennzeichnen;
eine neunte Kennzeichnungseinheit, die dazu dient, wenn die Anzahl von durch den ableitungspositiven Index als positiv angegebenen Hochfrequenz-QRS-Wellenformen 1 oder 2 ist, die Beschwerdesymptomdaten NEIN lauten, der Wellenformänderungs-Formindex die zweite Formkategorie ist und die positive Position die erste Kategorie ist, die den Hochfrequenz-QRS-Wellenformdaten entsprechende Aufmerksamkeitsstufe als eine neunte Aufmerksamkeitsstufe zu kennzeichnen; und
eine zehnte Kennzeichnungseinheit, die dazu dient, wenn die Anzahl von durch den ableitungspositiven Index als positiv angegebenen Hochfrequenz-QRS-Wellenformen 1 oder 2 ist, die Beschwerdesymptomdaten NEIN lauten, der Wellenformänderungs-Formindex die zweite Formkategorie ist und die positive Position die zweite Kategorie ist, die den Hochfrequenz-QRS-Wellenformdaten entsprechende Aufmerksamkeitsstufe als eine zehnte Aufmerksamkeitsstufe zu kennzeichnen.

5. Vorrichtung nach Anspruch 1, wobei das Modul (240) zur Bestimmung einer positiven Position umfasst:
eine erste Unterteilungseinheit, die dazu dient, wenn die durch den ableitungspositiven Index als positiv angegebene Hochfrequenz-QRS-Wellenform eine Brustwandableitung ist und die durch die Brustwandableitung ausgegebenen Hochfrequenz-QRS-Wellenformdaten mehrere Kombinationen von V1, V2, V3, V4, V5 und V6 sind, die positive Position gemäß der ersten voreingestellten Regel in die erste Kategorie zu unterteilen; und
eine zweite Unterteilungseinheit, die dazu dient, wenn die durch den ableitungspositiven Index als positiv angegebene Hochfrequenz-QRS-Wellenform eine Extremitätenableitung ist und die durch die Extremitätenableitung ausgegebenen Hochfrequenz-QRS-Wellenformdaten mehrere Kombinationen von I, II, III, aVL, aVF und aVR sind, die positive Position gemäß der ersten voreingestellten Regel in die zweite Kategorie zu unterteilen.

6. Computervorrichtung, umfassend einen Speicher und einen Prozessor, wobei der Speicher ein Computerprogramm speichert, wobei der Prozessor bei Ausführung des Computerprogramms ein Verfahren zur Analyse von Hochfrequenz-QRS-Komplex-Daten implementiert, welches umfasst:
Erfassen von Hochfrequenz-QRS-Wellenformdaten von einem Datenerfassungsmodul (210), die mittels mindestens einer Elektrokardiogramm-Ableitung ausgegeben werden, wobei die Hochfrequenz-QRS-Wellenformdaten Belastungszeitraum-Wellenformdaten umfassen, die Wellenformdaten während eines Zeitraums vor der Belastung, eines Zeitraums während der Belastung und eines Zeitraums nach der Belastung beinhalten;
Berechnen eines relativen Amplitudenabfallwerts und des Absolutwerts einer Amplitude anhand der Hochfrequenz-QRS-Wellenformdaten und mittels einer ersten Funktion, um einen ableitungspositiven Index zu bilden;
**dadurch gekennzeichnet, dass** ein Wellenformänderungs-Formindex über eine zweite Funktion anhand der Hochfrequenz-QRS-Wellenformdaten berechnet wird, wobei der Wellenformänderungs-Formindex dazu dient, eine Formkategorie anzugeben, zu der die Wellenformänderung gehört, wobei der Wellenformänderungs-Formindex eine erste Formkategorie, eine zweite Formkategorie und eine dritte Formkategorie umfasst; wobei die erste Formkategorie mindestens eines von einem U-Typ und einem L-Typ umfasst, die zweite Formkategorie mindestens eines von einem W-Typ, einem V-Typ und einem M-Typ umfasst und die dritte Formkategorie mindestens eines von einem flachen Typ und einem invertierten V-Typ umfasst;
Durchführen einer Analyse anhand des ableitungspositiven Index und des Wellenformänderungs-Formindex sowie mittels einer zweiten voreingestellten Regel, um eine den Hochfrequenz-QRS-Wellenformdaten entsprechende Aufmerksamkeitsstufe zu kennzeichnen; und
Unterteilen einer positiven Position in eine erste Kategorie oder eine zweite Kategorie gemäß einer ersten voreingestellten Regel basierend auf der Art und Menge der durch den ableitungspositiven Index als positiv angegebenen Hochfrequenz-QRS-Wellenformdaten;
Bestimmen der Aufmerksamkeitsstufe basierend auf:
(i) der Anzahl von durch den ableitungspositiven Index als positiv angegebenen Hochfrequenz-QRS-Wellenformen,
(ii) dem Wellenformänderungs-Formindex und
(iii) der unterteilten Kategorie der positiven Position,
um die den Hochfrequenz-QRS-Wellenformdaten entsprechende Aufmerksamkeitsstufe zu bestimmen;
wobei die zweite voreingestellte Regel unterschiedliche Aufmerksamkeitsstufen für unterschiedliche Analyseergebnisse definiert, die aus der kombinierten Analyse erhalten werden,
wobei das Berechnen des Wellenformänderungs-Formindex anhand der Hochfrequenz-QRS-Wellenformdaten und mittels der zweiten Funktion umfasst:
Erfassen von Festpunktdaten, an denen sich die Hochfrequenz-QRS-Wellenformdaten an einem Peak oder einem Tal befinden, wobei die Festpunktdaten einen Zeitwert und einen Effektivspannungswert (RMS) umfassen; wobei die Festpunktdaten Koordinaten eines Festpunkts in einem Elektrokardiogramm sind, wobei der Festpunkt an einer Peak- oder Talposition liegt; und
Eingeben der Festpunktdaten in die zweite Funktion, wobei die zweite Funktion einen Wellenformänderungs-Formindex ausgibt; und
wobei die zweite Funktion die Wellenformamplitude anhand der Amplitude zwischen benachbarten Festpunkten berechnet, die größte anhand der einzelnen Hochfrequenz-QRS-Wellenformdaten berechnete Wellenformamplitude auswählt, die Wellenformamplitude filtert, die kleiner als ein anhand der größten Wellenformamplitude berechneter Amplitudenschwellenwert ist, die der beibehaltenen Wellenformamplitude entsprechenden Festpunktdaten gemäß der zeitlichen Abfolge verbindet, um die Formfunktion zu erhalten, und den Wellenformänderungs-Formindex anhand der Formfunktion erhält.

7. Computervorrichtung nach Anspruch 6, wobei das Erfassen von Hochfrequenz-QRS-Wellenformdaten umfasst:
Erfassen der Hochfrequenz-QRS-Wellenformdaten für mindestens eine Abtastperiode, die mittels mindestens einer Elektrokardiogramm-Brustwandableitung und/oder -Extremitätenableitung ausgegeben werden, wobei die Hochfrequenz-QRS-Wellenformdaten durch Extrahieren von Daten in einem Intervall von 150 Hz bis 250 Hz aus einem Stromsignal erhalten werden, das durch ein an einem menschlichen Körper angebrachtes Elektrodenpad erfasst wird.

8. Computervorrichtung nach Anspruch 6, wobei das Berechnen eines relativen Amplitudenabfallwerts und des Absolutwerts einer Amplitude anhand der Hochfrequenz-QRS-Wellenformdaten und mittels einer ersten Funktion zur Bildung eines ableitungspositiven Index umfasst:
Abfangen von Belastungszeitraum-Wellenformdaten aus den Hochfrequenz-QRS-Wellenformdaten, Auswählen von Daten, bei denen der Effektivspannungswert (RMS) in den Belastungszeitraum-Wellenformdaten am größten ist, als einen ersten Referenzpunkt, und Auswählen eines Punkts, an dem der Effektivspannungswert (RMS) nach dem Zeitpunkt des ersten Referenzpunkts am kleinsten ist, als einen zweiten Referenzpunkt; und
Berechnen einer Differenz zwischen dem Effektivspannungswert (RMS) des ersten Referenzpunkts und dem Effektivspannungswert (RMS) des zweiten Referenzpunkts mittels der ersten Funktion, um einen Absolutwert der Amplitude zu erhalten, und Berechnen eines Verhältnisses des Absolutwerts der Amplitude zum Effektivspannungswert (RMS) des ersten Referenzpunkts, um einen relativen Amplitudenabfallwert zu erhalten, wobei der Absolutwert der Amplitude und der relative Amplitudenabfallwert einen ableitungspositiven Index bilden; wobei, wenn der Absolutwert und der relative Amplitudenabfallwert eine vorbestimmte Bedingung erfüllen, der ableitungspositive Index als positiv angegeben wird.

9. Computerlesbares Speichermedium, auf dem ein Computerprogramm gespeichert ist, wobei das Computerprogramm bei Ausführung durch einen Prozessor den Prozessor veranlasst, ein Verfahren zur Analyse von Hochfrequenz-QRS-Komplex-Daten durchzuführen, welches umfasst:
Erfassen von Hochfrequenz-QRS-Wellenformdaten von einem Datenerfassungsmodul (210), die mittels mindestens einer Elektrokardiogramm-Ableitung ausgegeben werden, wobei die Hochfrequenz-QRS-Wellenformdaten Belastungszeitraum-Wellenformdaten umfassen, die Wellenformdaten während eines Zeitraums vor der Belastung, eines Zeitraums während der Belastung und eines Zeitraums nach der Belastung beinhalten;
Berechnen eines relativen Amplitudenabfallwerts und des Absolutwerts einer Amplitude anhand der Hochfrequenz-QRS-Wellenformdaten und mittels einer ersten Funktion, um einen ableitungspositiven Index zu bilden;
**dadurch gekennzeichnet, dass** ein Wellenformänderungs-Formindex über eine zweite Funktion anhand der Hochfrequenz-QRS-Wellenformdaten berechnet wird, wobei der Wellenformänderungs-Formindex dazu dient, eine Formkategorie anzugeben, zu der die Wellenformänderung gehört, wobei der Wellenformänderungs-Formindex eine erste Formkategorie, eine zweite Formkategorie und eine dritte Formkategorie umfasst; wobei die erste Formkategorie mindestens eines von einem U-Typ und einem L-Typ umfasst, die zweite Formkategorie mindestens eines von einem W-Typ, einem V-Typ und einem M-Typ umfasst und die dritte Formkategorie mindestens eines von einem flachen Typ und einem invertierten V-Typ umfasst;
Durchführen einer Analyse anhand des ableitungspositiven Index und des Wellenformänderungs-Formindex sowie mittels einer zweiten voreingestellten Regel, um eine den Hochfrequenz-QRS-Wellenformdaten entsprechende Aufmerksamkeitsstufe zu kennzeichnen; und
Unterteilen einer positiven Position in eine erste Kategorie oder eine zweite Kategorie gemäß einer ersten voreingestellten Regel basierend auf der Art und Menge der durch den ableitungspositiven Index als positiv angegebenen Hochfrequenz-QRS-Wellenformdaten;
Bestimmen der Aufmerksamkeitsstufe basierend auf:
(i) der Anzahl von durch den ableitungspositiven Index als positiv angegebenen Hochfrequenz-QRS-Wellenformen,
(ii) dem Wellenformänderungs-Formindex und
(iii) der unterteilten Kategorie der positiven Position,
um die den Hochfrequenz-QRS-Wellenformdaten entsprechende Aufmerksamkeitsstufe zu bestimmen;
wobei die zweite voreingestellte Regel unterschiedliche Aufmerksamkeitsstufen für unterschiedliche Analyseergebnisse definiert, die aus der kombinierten Analyse erhalten werden,
wobei das Berechnen des Wellenformänderungs-Formindex anhand der Hochfrequenz-QRS-Wellenformdaten und mittels der zweiten Funktion umfasst:
Erfassen von Festpunktdaten, an denen sich die Hochfrequenz-QRS-Wellenformdaten an einem Peak oder einem Tal befinden, wobei die Festpunktdaten einen Zeitwert und einen Effektivspannungswert (RMS) umfassen; wobei die Festpunktdaten Koordinaten eines Festpunkts in einem Elektrokardiogramm sind, wobei der Festpunkt an einer Peak- oder Talposition liegt; und
Eingeben der Festpunktdaten in die zweite Funktion, wobei die zweite Funktion einen Wellenformänderungs-Formindex ausgibt; und
wobei die zweite Funktion die Wellenformamplitude anhand der Amplitude zwischen benachbarten Festpunkten berechnet, die größte anhand der einzelnen Hochfrequenz-QRS-Wellenformdaten berechnete Wellenformamplitude auswählt, die Wellenformamplitude filtert, die kleiner als ein anhand der größten Wellenformamplitude berechneter Amplitudenschwellenwert ist, die der beibehaltenen Wellenformamplitude entsprechenden Festpunktdaten gemäß der zeitlichen Abfolge verbindet, um die Formfunktion zu erhalten, und den Wellenformänderungs-Formindex anhand der Formfunktion erhält.

10. Computerlesbares Speichermedium nach Anspruch 9, wobei das Erfassen von Hochfrequenz-QRS-Wellenformdaten umfasst:
Erfassen der Hochfrequenz-QRS-Wellenformdaten für mindestens eine Abtastperiode, die mittels mindestens einer Elektrokardiogramm-Brustwandableitung und/oder -Extremitätenableitung ausgegeben werden, wobei die Hochfrequenz-QRS-Wellenformdaten durch Extrahieren von Daten in einem Intervall von 150 Hz bis 250 Hz aus einem Stromsignal erhalten werden, das durch ein an einem menschlichen Körper angebrachtes Elektrodenpad erfasst wird.

11. Computerlesbares Speichermedium nach Anspruch 9, wobei das Berechnen eines relativen Amplitudenabfallwerts und des Absolutwerts einer Amplitude anhand der Hochfrequenz-QRS-Wellenformdaten und mittels einer ersten Funktion zur Bildung eines ableitungspositiven Index umfasst:
Abfangen von Belastungszeitraum-Wellenformdaten aus den Hochfrequenz-QRS-Wellenformdaten, Auswählen von Daten, bei denen der Effektivspannungswert (RMS) in den Belastungszeitraum-Wellenformdaten am größten ist, als einen ersten Referenzpunkt, und Auswählen eines Punkts, an dem der Effektivspannungswert (RMS) nach dem Zeitpunkt des ersten Referenzpunkts am kleinsten ist, als einen zweiten Referenzpunkt; und
Berechnen einer Differenz zwischen dem Effektivspannungswert (RMS) des ersten Referenzpunkts und dem Effektivspannungswert (RMS) des zweiten Referenzpunkts mittels der ersten Funktion, um einen Absolutwert der Amplitude zu erhalten, und Berechnen eines Verhältnisses des Absolutwerts der Amplitude zum Effektivspannungswert (RMS) des ersten Referenzpunkts, um einen relativen Amplitudenabfallwert zu erhalten, wobei der Absolutwert der Amplitude und der relative Amplitudenabfallwert einen ableitungspositiven Index bilden; wobei, wenn der Absolutwert und der relative Amplitudenabfallwert eine vorbestimmte Bedingung erfüllen, der ableitungspositive Index als positiv angegeben wird.

## Revendications

1. Appareil pour analyser des données de complexe QRS haute fréquence, l'appareil comprenant :
un module d'acquisition de données (210), configuré pour acquérir des données de forme d'onde QRS haute fréquence, qui sont délivrées en sortie au moyen d'au moins une dérivation d'électrocardiogramme, dans lequel les données de forme d'onde QRS haute fréquence comprennent des données de forme d'onde de période d'effort qui incluent des données de forme d'onde pendant une période avant l'effort, une période pendant l'effort et une période après l'effort ;
un module de calcul d'indice positif de dérivation (220), configuré pour calculer une valeur relative de baisse d'amplitude et la valeur absolue d'une amplitude selon les données de forme d'onde QRS haute fréquence et au moyen d'une première fonction, de manière à former un indice positif de dérivation ;
un module de calcul d'indice de forme de changement de forme d'onde (230), **caractérisé en ce que** le module de calcul d'indice de forme de changement de forme d'onde est configuré pour calculer un indice de forme de changement de forme d'onde par l'intermédiaire d'une deuxième fonction selon les données de forme d'onde QRS haute fréquence, l'indice de forme de changement de forme d'onde étant configuré pour indiquer une catégorie de forme à laquelle appartient le changement de forme d'onde, dans lequel l'indice de forme de changement de forme d'onde comprend une première catégorie de forme, une deuxième catégorie de forme et une troisième catégorie de forme ; dans lequel la première catégorie de forme comprend au moins l'un d'un type U et d'un type L, la deuxième catégorie de forme comprend au moins l'un d'un type W, d'un type V et d'un type M, et la troisième catégorie de forme comprend au moins l'un d'un type plat et d'un type V inversé ;
un module d'étiquetage (250), configuré pour effectuer une analyse selon l'indice positif de dérivation et l'indice de forme de changement de forme d'onde et au moyen d'une deuxième règle prédéfinie, de manière à étiqueter un niveau d'attention correspondant aux données de forme d'onde QRS haute fréquence ; et
un module de détermination de position positive (240), configuré pour diviser une position positive en une première catégorie ou une deuxième catégorie selon une première règle prédéfinie sur la base de la nature et de la quantité des données de forme d'onde QRS haute fréquence indiquées comme positives par l'indice positif de dérivation ;
dans lequel le module d'étiquetage est configuré pour déterminer le niveau d'attention sur la base de :
(i) le nombre de formes d'onde QRS haute fréquence indiquées comme positives par l'indice positif de dérivation,
(ii) l'indice de forme de changement de forme d'onde, et
(iii) la catégorie divisée de la position positive,
de manière à déterminer le niveau d'attention correspondant aux données de forme d'onde QRS haute fréquence ;
dans lequel la deuxième règle prédéfinie définit différents niveaux d'attention pour différents résultats d'analyse obtenus à partir de ladite analyse combinée,
dans lequel le calcul de l'indice de forme de changement de forme d'onde selon les données de forme d'onde QRS haute fréquence et au moyen de la deuxième fonction comprend :
l'acquisition de données de point fixe selon lesquelles les données de forme d'onde QRS haute fréquence sont situées au niveau d'un pic ou d'un creux, les données de point fixe comprenant une valeur de temps et une valeur de tension efficace (RMS) ; les données de point fixe étant des coordonnées d'un point fixe dans un électrocardiogramme, le point fixe étant à une position de pic ou de creux ; et
l'entrée des données de point fixe dans la deuxième fonction, la deuxième fonction délivrant en sortie un indice de forme de changement de forme d'onde ; et
dans lequel la deuxième fonction calcule l'amplitude de forme d'onde selon l'amplitude entre des points fixes adjacents, choisit la plus grande amplitude de forme d'onde calculée selon les données uniques de forme d'onde QRS haute fréquence, filtre l'amplitude de forme d'onde plus petite qu'un seuil d'amplitude calculé selon la plus grande amplitude de forme d'onde, connecte les données de point fixe correspondant à l'amplitude de forme d'onde retenue selon la séquence temporelle pour obtenir la fonction de forme, et obtient l'indice de forme de changement de forme d'onde selon la fonction de forme.

2. Appareil selon la revendication 1, dans lequel le module d'acquisition de données (210) est en outre utilisé pour acquérir les données de forme d'onde QRS haute fréquence pendant au moins une période d'échantillonnage, qui sont délivrées en sortie au moyen d'au moins une dérivation précordiale et/ou dérivation des membres d'électrocardiogramme, les données de forme d'onde QRS haute fréquence étant obtenues par extraction de données dans un intervalle de 150 Hz à 250 Hz à partir d'un signal de courant acquis par un tampon d'électrode appliqué à un corps humain.

3. Appareil selon la revendication 1, dans lequel le module de calcul d'indice positif de dérivation (220) comprend :
une unité de choix de point de référence, utilisée pour intercepter des données de forme d'onde de période d'effort à partir des données de forme d'onde QRS haute fréquence, choisir des données auxquelles la valeur de tension efficace (RMS) est la plus grande dans les données de forme d'onde de période d'effort en tant que premier point de référence, et choisir un point auquel la valeur de tension efficace (RMS) est la plus petite après le moment du premier point de référence en tant que deuxième point de référence ; et
une unité de calcul d'indice positif de dérivation, utilisée pour calculer une différence entre la valeur de tension efficace (RMS) du premier point de référence et la valeur de tension efficace (RMS) du deuxième point de référence au moyen de la première fonction pour obtenir une valeur absolue de l'amplitude, et calculer un rapport de la valeur absolue de l'amplitude à la valeur de tension efficace (RMS) du premier point de référence pour obtenir une valeur relative de baisse d'amplitude, la valeur absolue de l'amplitude et la valeur relative de baisse d'amplitude constituant un indice positif de dérivation ; dans lequel si la valeur absolue et la valeur relative de baisse d'amplitude satisfont à une condition prédéterminée, l'indice positif de dérivation est indiqué comme positif.

4. Appareil selon la revendication 1, dans lequel le module d'étiquetage (250) comprend :
une première unité d'étiquetage, utilisée pour, lorsque le nombre de formes d'onde QRS haute fréquence indiquées comme positives par l'indice positif de dérivation est supérieur à 5, que l'indice de forme de changement de forme d'onde est la première catégorie de forme, et que la position positive est la première catégorie, étiqueter le niveau d'attention correspondant aux données de forme d'onde QRS haute fréquence en tant que premier niveau d'attention ;
une deuxième unité d'étiquetage, utilisée pour, lorsque le nombre de formes d'onde QRS haute fréquence indiquées comme positives par l'indice positif de dérivation est supérieur à 5, que l'indice de forme de changement de forme d'onde est la deuxième catégorie de forme, et que la position positive est la deuxième catégorie, étiqueter le niveau d'attention correspondant aux données de forme d'onde QRS haute fréquence en tant que deuxième niveau d'attention ;
une troisième unité d'étiquetage, utilisée pour, lorsque le nombre de formes d'onde QRS haute fréquence indiquées comme positives par l'indice positif de dérivation est supérieur ou égal à 3 et non supérieur à 5, que les données de symptôme d'inconfort sont OUI, que l'indice de forme de changement de forme d'onde est la première catégorie de forme, et que la position positive est la première catégorie, étiqueter le niveau d'attention correspondant aux données de forme d'onde QRS haute fréquence en tant que troisième niveau d'attention ;
une quatrième unité d'étiquetage, utilisée pour, lorsque le nombre de formes d'onde QRS haute fréquence indiquées comme positives par l'indice positif de dérivation est supérieur ou égal à 3 et non supérieur à 5, que les données de symptôme d'inconfort sont OUI, que l'indice de forme de changement de forme d'onde est la première catégorie de forme, et que la position positive est la deuxième catégorie, étiqueter le niveau d'attention correspondant aux données de forme d'onde QRS haute fréquence en tant que quatrième niveau d'attention ;
une cinquième unité d'étiquetage, utilisée pour, lorsque le nombre de formes d'onde QRS haute fréquence indiquées comme positives par l'indice positif de dérivation est supérieur ou égal à 3 et non supérieur à 5, que les données de symptôme d'inconfort sont NON, que l'indice de forme de changement de forme d'onde est la deuxième catégorie de forme, et que la position positive est la première catégorie, étiqueter le niveau d'attention correspondant aux données de forme d'onde QRS haute fréquence en tant que cinquième niveau d'attention ;
une sixième unité d'étiquetage, utilisée pour, lorsque le nombre de formes d'onde QRS haute fréquence indiquées comme positives par l'indice positif de dérivation est supérieur ou égal à 3 et non supérieur à 5, que les données de symptôme d'inconfort sont NON, que l'indice de forme de changement de forme d'onde est la deuxième catégorie de forme, et que la position positive est la deuxième catégorie, étiqueter le niveau d'attention correspondant aux données de forme d'onde QRS haute fréquence en tant que sixième niveau d'attention ;
une septième unité d'étiquetage, utilisée pour, lorsque le nombre de formes d'onde QRS haute fréquence indiquées comme positives par l'indice positif de dérivation est égal à 1 ou 2, que les données de symptôme d'inconfort sont OUI, que l'indice de forme de changement de forme d'onde est la première catégorie de forme, et que la position positive est la première catégorie, étiqueter le niveau d'attention correspondant aux données de forme d'onde QRS haute fréquence en tant que septième niveau d'attention ;
une huitième unité d'étiquetage, utilisée pour, lorsque le nombre de formes d'onde QRS haute fréquence indiquées comme positives par l'indice positif de dérivation est égal à 1 ou 2, que les données de symptôme d'inconfort sont OUI, que l'indice de forme de changement de forme d'onde est la première catégorie de forme, et que la position positive est la deuxième catégorie, étiqueter le niveau d'attention correspondant aux données de forme d'onde QRS haute fréquence en tant que huitième niveau d'attention ;
une neuvième unité d'étiquetage, utilisée pour, lorsque le nombre de formes d'onde QRS haute fréquence indiquées comme positives par l'indice positif de dérivation est égal à 1 ou 2, que les données de symptôme d'inconfort sont NON, que l'indice de forme de changement de forme d'onde est la deuxième catégorie de forme, et que la position positive est la première catégorie, étiqueter le niveau d'attention correspondant aux données de forme d'onde QRS haute fréquence en tant que neuvième niveau d'attention ; et
une dixième unité d'étiquetage, utilisée pour, lorsque le nombre de formes d'onde QRS haute fréquence indiquées comme positives par l'indice positif de dérivation est égal à 1 ou 2, que les données de symptôme d'inconfort sont NON, que l'indice de forme de changement de forme d'onde est la deuxième catégorie de forme, et que la position positive est la deuxième catégorie, étiqueter le niveau d'attention correspondant aux données de forme d'onde QRS haute fréquence en tant que dixième niveau d'attention.

5. Appareil selon la revendication 1, dans lequel le module de détermination de position positive (240) comprend :
une première unité de division, utilisée pour, lorsque la forme d'onde QRS haute fréquence indiquée comme positive par l'indice positif de dérivation est une dérivation précordiale, et que les données de forme d'onde QRS haute fréquence délivrées en sortie par la dérivation précordiale sont une pluralité de combinaisons de V1, V2, V3, V4, V5 et V6, diviser la position positive en la première catégorie selon la première règle prédéfinie ; et
une deuxième unité de division, utilisée pour, lorsque la forme d'onde QRS haute fréquence indiquée comme positive par l'indice positif de dérivation est une dérivation des membres, et que les données de forme d'onde QRS haute fréquence délivrées en sortie par la dérivation des membres sont une pluralité de combinaisons de I, II, III, aVL, aVF et aVR, diviser la position positive en la deuxième catégorie selon la première règle prédéfinie.

6. Dispositif informatique, comprenant une mémoire et un processeur, la mémoire stockant un programme informatique, dans lequel le processeur, lors de l'exécution du programme informatique, met en œuvre un procédé d'analyse de données de complexe QRS haute fréquence, qui comprend :
l'acquisition de données de forme d'onde QRS haute fréquence à partir d'un module d'acquisition de données (210), qui sont délivrées en sortie au moyen d'au moins une dérivation d'électrocardiogramme, dans lequel les données de forme d'onde QRS haute fréquence comprennent des données de forme d'onde de période d'effort qui incluent des données de forme d'onde pendant une période avant l'effort, une période pendant l'effort et une période après l'effort ;
le calcul d'une valeur relative de baisse d'amplitude et de la valeur absolue d'une amplitude selon les données de forme d'onde QRS haute fréquence et au moyen d'une première fonction, de manière à former un indice positif de dérivation ;
**caractérisé en ce qu'**un indice de forme de changement de forme d'onde est calculé par l'intermédiaire d'une deuxième fonction selon les données de forme d'onde QRS haute fréquence, dans lequel l'indice de forme de changement de forme d'onde est utilisé pour indiquer une catégorie de forme à laquelle appartient le changement de forme d'onde, dans lequel l'indice de forme de changement de forme d'onde comprend une première catégorie de forme, une deuxième catégorie de forme et une troisième catégorie de forme ; dans lequel la première catégorie de forme comprend au moins l'un d'un type U et d'un type L, la deuxième catégorie de forme comprend au moins l'un d'un type W, d'un type V et d'un type M, et la troisième catégorie de forme comprend au moins l'un d'un type plat et d'un type V inversé ;
la réalisation d'une analyse selon l'indice positif de dérivation et l'indice de forme de changement de forme d'onde et au moyen d'une deuxième règle prédéfinie, de manière à étiqueter un niveau d'attention correspondant aux données de forme d'onde QRS haute fréquence ; et
la division d'une position positive en une première catégorie ou une deuxième catégorie selon une première règle prédéfinie sur la base de la nature et de la quantité des données de forme d'onde QRS haute fréquence indiquées comme positives par l'indice positif de dérivation ;
la détermination du niveau d'attention sur la base de :
(i) le nombre de formes d'onde QRS haute fréquence indiquées comme positives par l'indice positif de dérivation,
(ii) l'indice de forme de changement de forme d'onde, et
(iii) la catégorie divisée de la position positive,
de manière à déterminer le niveau d'attention correspondant aux données de forme d'onde QRS haute fréquence ;
dans lequel la deuxième règle prédéfinie définit différents niveaux d'attention pour différents résultats d'analyse obtenus à partir de ladite analyse combinée,
dans lequel le calcul de l'indice de forme de changement de forme d'onde selon les données de forme d'onde QRS haute fréquence et au moyen de la deuxième fonction comprend :
l'acquisition de données de point fixe selon lesquelles les données de forme d'onde QRS haute fréquence sont situées au niveau d'un pic ou d'un creux, les données de point fixe comprenant une valeur de temps et une valeur de tension efficace (RMS) ; les données de point fixe étant des coordonnées d'un point fixe dans un électrocardiogramme, le point fixe étant à une position de pic ou de creux ; et
l'entrée des données de point fixe dans la deuxième fonction, la deuxième fonction délivrant en sortie un indice de forme de changement de forme d'onde ; et
dans lequel la deuxième fonction calcule l'amplitude de forme d'onde selon l'amplitude entre des points fixes adjacents, choisit la plus grande amplitude de forme d'onde calculée selon les données uniques de forme d'onde QRS haute fréquence, filtre l'amplitude de forme d'onde plus petite qu'un seuil d'amplitude calculé selon la plus grande amplitude de forme d'onde, connecte les données de point fixe correspondant à l'amplitude de forme d'onde retenue selon la séquence temporelle pour obtenir la fonction de forme, et obtient l'indice de forme de changement de forme d'onde selon la fonction de forme.

7. Dispositif informatique selon la revendication 6, dans lequel l'acquisition de données de forme d'onde QRS haute fréquence comprend :
l'acquisition des données de forme d'onde QRS haute fréquence pendant au moins une période d'échantillonnage, qui sont délivrées en sortie au moyen d'au moins une dérivation précordiale et/ou dérivation des membres d'électrocardiogramme, les données de forme d'onde QRS haute fréquence étant obtenues par extraction de données dans un intervalle de 150 Hz à 250 Hz à partir d'un signal de courant acquis par un tampon d'électrode appliqué à un corps humain.

8. Dispositif informatique selon la revendication 6, dans lequel le calcul d'une valeur relative de baisse d'amplitude et de la valeur absolue d'une amplitude selon les données de forme d'onde QRS haute fréquence et au moyen d'une première fonction, de manière à former un indice positif de dérivation comprend :
l'interception de données de forme d'onde de période d'effort à partir des données de forme d'onde QRS haute fréquence, le choix de données auxquelles la valeur de tension efficace (RMS) est la plus grande dans les données de forme d'onde de période d'effort en tant que premier point de référence, et le choix d'un point auquel la valeur de tension efficace (RMS) est la plus petite après le moment du premier point de référence en tant que deuxième point de référence ; et
le calcul d'une différence entre la valeur de tension efficace (RMS) du premier point de référence et la valeur de tension efficace (RMS) du deuxième point de référence au moyen de la première fonction pour obtenir une valeur absolue de l'amplitude, et le calcul d'un rapport de la valeur absolue de l'amplitude à la valeur de tension efficace (RMS) du premier point de référence pour obtenir une valeur relative de baisse d'amplitude, la valeur absolue de l'amplitude et la valeur relative de baisse d'amplitude constituant un indice positif de dérivation ; dans lequel si la valeur absolue et la valeur relative de baisse d'amplitude satisfont à une condition prédéterminée, l'indice positif de dérivation est indiqué comme positif.

9. Support de stockage lisible par ordinateur sur lequel est stocké un programme informatique, qui, lorsqu'il est exécuté par un processeur, amène le processeur à réaliser un procédé d'analyse de données de complexe QRS haute fréquence, qui comprend :
l'acquisition de données de forme d'onde QRS haute fréquence à partir d'un module d'acquisition de données (210), qui sont délivrées en sortie au moyen d'au moins une dérivation d'électrocardiogramme, dans lequel les données de forme d'onde QRS haute fréquence comprennent des données de forme d'onde de période d'effort qui incluent des données de forme d'onde pendant une période avant l'effort, une période pendant l'effort et une période après l'effort ;
le calcul d'une valeur relative de baisse d'amplitude et de la valeur absolue d'une amplitude selon les données de forme d'onde QRS haute fréquence et au moyen d'une première fonction, de manière à former un indice positif de dérivation ;
**caractérisé en ce qu'**un indice de forme de changement de forme d'onde est calculé par l'intermédiaire d'une deuxième fonction selon les données de forme d'onde QRS haute fréquence, dans lequel l'indice de forme de changement de forme d'onde est utilisé pour indiquer une catégorie de forme à laquelle appartient le changement de forme d'onde, dans lequel l'indice de forme de changement de forme d'onde comprend une première catégorie de forme, une deuxième catégorie de forme et une troisième catégorie de forme ; dans lequel la première catégorie de forme comprend au moins l'un d'un type U et d'un type L, la deuxième catégorie de forme comprend au moins l'un d'un type W, d'un type V et d'un type M, et la troisième catégorie de forme comprend au moins l'un d'un type plat et d'un type V inversé ;
la réalisation d'une analyse selon l'indice positif de dérivation et l'indice de forme de changement de forme d'onde et au moyen d'une deuxième règle prédéfinie, de manière à étiqueter un niveau d'attention correspondant aux données de forme d'onde QRS haute fréquence ; et
la division d'une position positive en une première catégorie ou une deuxième catégorie selon une première règle prédéfinie sur la base de la nature et de la quantité des données de forme d'onde QRS haute fréquence indiquées comme positives par l'indice positif de dérivation ;
la détermination du niveau d'attention sur la base de :
(i) le nombre de formes d'onde QRS haute fréquence indiquées comme positives par l'indice positif de dérivation,
(ii) l'indice de forme de changement de forme d'onde, et
(iii) la catégorie divisée de la position positive,
de manière à déterminer le niveau d'attention correspondant aux données de forme d'onde QRS haute fréquence ;
dans lequel la deuxième règle prédéfinie définit différents niveaux d'attention pour différents résultats d'analyse obtenus à partir de ladite analyse combinée,
dans lequel le calcul de l'indice de forme de changement de forme d'onde selon les données de forme d'onde QRS haute fréquence et au moyen de la deuxième fonction comprend :
l'acquisition de données de point fixe selon lesquelles les données de forme d'onde QRS haute fréquence sont situées au niveau d'un pic ou d'un creux, les données de point fixe comprenant une valeur de temps et une valeur de tension efficace (RMS) ; les données de point fixe étant des coordonnées d'un point fixe dans un électrocardiogramme, le point fixe étant à une position de pic ou de creux ; et
l'entrée des données de point fixe dans la deuxième fonction, la deuxième fonction délivrant en sortie un indice de forme de changement de forme d'onde ; et
dans lequel la deuxième fonction calcule l'amplitude de forme d'onde selon l'amplitude entre des points fixes adjacents, choisit la plus grande amplitude de forme d'onde calculée selon les données uniques de forme d'onde QRS haute fréquence, filtre l'amplitude de forme d'onde plus petite qu'un seuil d'amplitude calculé selon la plus grande amplitude de forme d'onde, connecte les données de point fixe correspondant à l'amplitude de forme d'onde retenue selon la séquence temporelle pour obtenir la fonction de forme, et obtient l'indice de forme de changement de forme d'onde selon la fonction de forme.

10. Support de stockage lisible par ordinateur selon la revendication 9, dans lequel l'acquisition de données de forme d'onde QRS haute fréquence comprend :
l'acquisition des données de forme d'onde QRS haute fréquence pendant au moins une période d'échantillonnage, qui sont délivrées en sortie au moyen d'au moins une dérivation précordiale et/ou dérivation des membres d'électrocardiogramme, les données de forme d'onde QRS haute fréquence étant obtenues par extraction de données dans un intervalle de 150 Hz à 250 Hz à partir d'un signal de courant acquis par un tampon d'électrode appliqué à un corps humain.

11. Support de stockage lisible par ordinateur selon la revendication 9, dans lequel le calcul d'une valeur relative de baisse d'amplitude et de la valeur absolue d'une amplitude selon les données de forme d'onde QRS haute fréquence et au moyen d'une première fonction, de manière à former un indice positif de dérivation comprend :
l'interception de données de forme d'onde de période d'effort à partir des données de forme d'onde QRS haute fréquence, le choix de données auxquelles la valeur de tension efficace (RMS) est la plus grande dans les données de forme d'onde de période d'effort en tant que premier point de référence, et le choix d'un point auquel la valeur de tension efficace (RMS) est la plus petite après le moment du premier point de référence en tant que deuxième point de référence ; et
le calcul d'une différence entre la valeur de tension efficace (RMS) du premier point de référence et la valeur de tension efficace (RMS) du deuxième point de référence au moyen de la première fonction pour obtenir une valeur absolue de l'amplitude, et le calcul d'un rapport de la valeur absolue de l'amplitude à la valeur de tension efficace (RMS) du premier point de référence pour obtenir une valeur relative de baisse d'amplitude, la valeur absolue de l'amplitude et la valeur relative de baisse d'amplitude constituant un indice positif de dérivation ; dans lequel si la valeur absolue et la valeur relative de baisse d'amplitude satisfont à une condition prédéterminée, l'indice positif de dérivation est indiqué comme positif.
